# EUROPEAN PATENT SPECIFICATION

(11) **EP 2 955 223 B1**
(45) Date of publication and mention of the grant of the patent: **18.12.2019**
(21) Application number: 14749207.8
(22) Date of filing: 10.02.2014
(51) Int. Cl.: C12N 5/10, A61K 35/12, A61P 7/00, A61K 35/19, A61K 35/28, C12N 5/078

(54) **PRODUCTION METHODS FOR MEGAKARYOCYTES AND PLATELETS**
HERSTELLUNGSVERFAHREN FÜR MEGAKARYOZYTEN UND BLUTPLÄTTCHEN
PROCÉDÉS DE PRODUCTION DE MÉGACARYOCYTES ET DE PLAQUETTES

(30) Priority: 08.02.2013 JP 2013023013
(43) Date of publication of application: 16.12.2015
(73) Proprietor: Kyoto University, Kyoto-shi, Kyoto 606-8501 (JP)
(72) Inventor: ETO, Koji, Kyoto-shi Kyoto 606-8501 (JP)
(74) Representative: Mewburn Ellis LLP
(86) International application number: PCT/JP2014/053087
(87) International publication number: WO 2014/123242

(56) References cited:
- WO-A1-2011/034073
- WO-A1-2012/157586
- JP-A- 2002 526 093
- KLUTER H. ET AL.: 'Impact of buffy coat storage on the generation of inflammatory cytokines and platelet activation' TRANSFUSION vol. 37, 01 April 1997, pages 362 - 367, XP055272486 DOI: 10.1046/J.1537-2995.1997.37497265335.X
- DORE LOUIS C. ET AL.: 'Transcription factor networks in erythroid cell and megakaryocyte development' BLOOD vol. 118, no. 2, 14 July 2011, pages 231 - 239, XP055272494 DOI: 10.1182/BLOOD-2011-04-285981
- TAKAYAMA NAOYA: 'Platelet Production System Using an Immortalized Megakaryocyte Cell Line Derived From Human Pluripotent Stem Cells' AMERICAN SOCIETY OF HEMATOLOGY 10 December 2011, XP008180270 Retrieved from the Internet: <URL:http://www.reuters.com/article/2011/12 /10/idUS70817+10-Dec-2011+PRN20111210> [retrieved on 2014-04-24]

### SEQUENCE LISTING

<110> KYOTO UNIVERSITY
<120> Method for producing megakaryocyte and platelet
<130> K0875AFP0001
<150> JP 2013-023013
   <151> 2013-02-08
<160> 6
<170> PatentIn version 3.1
<210> 1
   <211> 20
   <212> PRT
   <213> Artificial
<220>
   <223> A synthesized caspase inhibitor peptide.
<220>
   <221> MOD_RES
   <222> (1)..(1)
   <223> ACETYLATION
<220>
   <221> MOD_RES
   <222> (20)..(20)
   <223> Acetaldehyde-modified aspartic acid.
<400> 1
   Ala Ala Val Ala Leu Leu Pro Ala Val Leu Leu Ala Leu Leu Ala Pro
   1               5                   10                  15
   Tyr Val Ala Asp
               20
<210> 2
   <211> 20
   <212> PRT
   <213> Artificial
<220>
   <223> A synthesized caspase inhibitor peptide.
<220>
   <221> MOD_RES
   <222> (1)..(1)
   <223> ACETYLATION
<220>
   <221> MOD_RES
   <222> (20)..(20)
   <223> Acetaldehyde-modified aspartic acid.
<400> 2
   Ala Ala Val Ala Leu Leu Pro Ala Val Leu Leu Ala Leu Leu Ala Pro
   1               5                   10                  15
   Asp Glu Val Asp
               20
<210> 3
   <211> 20
   <212> PRT
   <213> Artificial
<220>
   <223> A synthesized caspase inhibitor peptide.
<220>
   <221> MOD_RES
   <222> (1)..(1)
   <223> ACETYLATION
<220>
   <221> MOD_RES
   <222> (20)..(20)
   <223> Acetaldehyde-modified aspartic acid.
<400> 3
   Ala Ala Val Ala Leu Leu Pro Ala Val Leu Leu Ala Leu Leu Ala Pro
   1               5                   10                  15
   Leu Glu Val Asp
               20
<210> 4
   <211> 20
   <212> PRT
   <213> Artificial
<220>
   <223> A synthesized caspase inhibitor peptide.
<220>
   <221> MOD_RES
   <222> (1)..(1)
   <223> ACETYLATION
<220>
   <221> MOD_RES
   <222> (20)..(20)
   <223> Acetaldehyde-modified aspartic acid.
<400> 4
   Ala Ala Val Ala Leu Leu Pro Ala Val Leu Leu Ala Leu Leu Ala Pro
   1               5                   10                  15
   Ile Glu Thr Asp
               20
<210> 5
   <211> 20
   <212> PRT
   <213> Artificial
<220>
   <223> A synthesized caspase inhibitor peptide.
<220>
   <221> MOD_RES
   <222> (1)..(1)
   <223> ACETYLATION
<220>
   <221> MOD_RES
   <222> (20)..(20)
   <223> Acetaldehyde-modified aspartic acid.
<400> 5
   Ala Ala Val Ala Leu Leu Pro Ala Val Leu Leu Ala Leu Leu Ala Pro
   1               5                   10                  15
   Leu Glu His Asp
               20
<210> 6
   <211> 4
   <212> PRT
   <213> Artificial
<220>
   <223> A synthesized caspase inhibitor peptide.
<220>
   <221> MOD_RES
   <222> (1)..(1)
   <223> Benzyloxycarbonyl-modified aspartic acid.
<220>
   <221> MOD_RES
   <222> (4)..(4)
   <223> Fluoromethylketone-modified aspartic acid.
<400> 6
   Asp Glu Val Asp
   1

## Description

### Technical Field

The present invention relates to a method of producing megakaryocytes and platelets from hematopoietic progenitor cells. Also described herein is a method of selecting hematopoietic progenitor cells suitable for producing megakaryocytes and others.

### Background Art

A large number of blood cells are required for treating blood related diseases and surgical therapy. Of the blood cells, platelets, which are essential for coagulation of blood and blood stanching, are one of the particularly important blood cells. Platelets are often required for treating e.g., leukemia, bone marrow transplantation and anticancer therapy, and stable supply of the platelets is highly needed. Up to present, platelets have been obtained from blood collected through a blood donation system. Other than this method, platelets have been supplied by a method of administering a preparation of a TPO-like similar structure (mimetics) and a method of differentiating from umbilical cord blood or bone marrow cells to megakaryocytes. Recently, a technique for preparing blood cells such as platelets by inducing *in vitro* differentiation of pluripotent stem cells such as ES cells or iPS cells has been developed.

The inventors have established a technique for obtaining megakaryocytes and platelets through induction of differentiation of human ES cells or iPS cells and demonstrated effectiveness of pluripotent stem cells as a source of platelets (Patent Literature 1, Non-Patent Literature 1 and Patent Literature 2).

The inventors further found a method for establishing an immortalized megakaryocytic progenitor cell line from stem cells to solve a problem relating to the amount of platelets etc. prepared from stem cells, and developed a technique important for preparing a large amount of platelets etc. *in vitro* (Patent Literature 3). At this time, they succeeded in maturing megakaryocytes by forcibly expressing an apoptosis suppression gene, Bcl-xl, in a step of producing megakaryocytes (Patent Literature 4).

In a living body, megakaryocytes form pseudopods called proplatelets and release platelets by fragmenting the cytoplasm of the pseudopods. It is considered that megakaryocytes are multinucleated by endomitosis before they release platelets. The endomitosis of a megakaryocyte is multipolar mitotic division accompanying neither cleavage furrow formation nor spindle extension and caused by abnormality in nuclear division and cytoplasm division. As a result, a cell containing some lobed nuclei is formed. Such endomitosis is repeatedly performed to induce multinucleated megakaryocytes.

A large number of research results have been reported regarding multinucleation of megakaryocytes up to present. Lordier et al. (Non-Patent Literature 1) found that, in the endomitosis of megakaryocytes, a cleavage furrow is formed but localization of non-muscle cell myosin II in a contractile ring is not found, causing defective contractile ring formation and defective spindle extension. It was reported that abnormality of the contractile ring and spindle extension is more significantly observed by inhibiting the activities of RhoA and Rock (Non-Patent Literature 2). RhoA is accumulated in cleavage furrow and accelerates activation of effector factors including Rho kinase (Rock), citron kinase, LIM kinase and mDia/formins. These results suggest that endomitosis of megakaryocytes is accelerated by inhibiting the activity of factors such as RhoA and Rock involved in formation of a contractile ring. In addition, it is also reported that if the intensity of a signal from Rho located downstream of integrin alpha2/beta1 increases, proplatelet formation of immature and not multinucleated megakaryocytes is inhibited.

It is reported that a transcription factor, i.e., all-trans retinoic acid (ATRA) and valproic acid known to serve as a histone deacetylation enzyme inhibitor are involved in differentiation of megakaryocytes. Schweinfurth et al. have found that if immature megakaryocytes are treated with all-trans retinoic acid or valproic acid, multinucleation of the megakaryocytes is accelerated (Non-Patent Literature 3). In addition, it is also reported that multinucleation of megakaryocytes is accelerated by knocking down a cancer suppressor gene product, p53 (Non-Patent Literature 4).

Other than the aforementioned reports, as a report on the effect on the differentiation process of megakaryocytes, it is described that if immature megakaryocytes are cultured at a temperature higher than general culture temperature, i.e., 39°C, induction of multinucleated matured megakaryocytes and formation of proplatelets are accelerated (Non-Patent Literature 5).

### Citation List

### Patent Literature

Patent Literature 1: WO2008/041370
Patent Literature 2: WO2009/122747
Patent Literature 3: WO2011/034073
Patent Literature 4: WO2012/157586

### Non Patent Literature

Non-Patent Literature 1: Takayama et al., Blood, 111: 5298-5306 2008
Non-Patent Literature 2: Lordier et al., Blood, 112: 3164-3174 2008
Non-Patent Literature 3: Schweinfurth et al., Platelets, 21: 648-657 2010
Non-Patent Literature 4: Fuhrken et al., J. Biol. Chem., 283: 15589-15600 2008
Non-Patent Literature 5: Proulx et al., Biotechnol. Bioeng., 88: 675-680 2004

### Summary of Invention

### Technical Problem

The present inventors found that in order to produce a platelet preparation, it is necessary to establish a megakaryocytic cell line that stably and largely produces platelets (platelets having activity such as blood stanching, *in vivo,* and characterized by CD42b+), which are more functional than those produced by methods known in the art. To attain this, they thought that it is necessary to further mature the megakaryocytic cell line obtained by a method known in the art.

Then, described herein as an object to provide a method for maturing megakaryocytes by terminating proliferation in place of simply increasing the number of megakaryocytes; and a method for selecting materials suitable for producing such megakaryocytes, and others.

### Solution to Problem

To attain the object described herein, the present inventors prepared hematopoietic progenitor cells from pluripotent stem cells (e.g., ES cells, iPS cells) and tried to find difference between the hematopoietic progenitor cells suitable and not suitable for establishing megakaryocytes. They further tried to arrest forced expression of a gene required for inducing differentiation of hematopoietic progenitor cells into megakaryocytes, in order to mature the megakaryocytes.

The inventors repeated the aforementioned trial and have found that KLF1 and FLI1 serve as markers for hematopoietic progenitor cells from which megakaryocytes are easily established.

They have further found that the function of megakaryocytes can be maintained by arresting forced expression of essential genes in establishing megakaryocytes. They have further found that megakaryocytes that stop proliferation by arresting expression of the genes as mentioned above more efficiently produce functional platelets. Based on the findings, the present invention was achieved.

The invention is defined in the appended claims.

More specifically, the present disclosure relates to the following.
[1] A method for producing megakaryocytes from hematopoietic progenitor cells, comprising the following (i) and (ii) steps:
   (i) forcibly expressing BCL-XL gene and an C-MYC gene in hematopoietic progenitor cells and culturing the cells, and
   (ii) arresting forced expression of the BCL-XL gene and the C-MYC gene in the cells obtained in the step (i) and culturing the cells.
[2] The method according to [1], wherein, in the step (i), BMI 1 is further forcibly expressed in the hematopoietic progenitor cells; and in the step (ii), the forced expression of the BMI1 is arrested and the culture is performed.
[3] The method according to [2], wherein, the step (i) is a step of forcibly expressing the C-MYC gene and the BMI1 in hematopoietic progenitor cells, and thereafter, further forcibly expressing the BCL-XL gene in the cells.
[4] The method according to [3], wherein, in the step (i), the hematopoietic progenitor cells are cultured for at least 28 days while the C-MYC gene and the BMI1 are forcibly expressed in the hematopoietic progenitor cells, and thereafter, further the BCL-XL gene is forcibly expressed in the cells.
[5] The method according to any one of [1] to [4], wherein, in the steps (i) and (ii), the cells are cultured on C3H10T1/2 cells in a culture solution containing TPO.
[6] The method according to [5], wherein in the steps (i) and (ii), the culture is performed in the culture solution further containing SCF.
[7] The method according to any one of [1] to [6], wherein the forced expression of the genes is performed by a drug responsive vector.
[8] The method according to any one of [1] to [7], wherein the hematopoietic progenitor cells are cells differentiation-induced from pluripotent stem cells.
[9] The method according to [8], wherein the hematopoietic progenitor cells are cells differentiation-induced from pluripotent stem cells, comprising culturing the pluripotent stem cells on C3H10T1/2 cells in a culture solution containing VEGF, in the differentiation induction.
[10] The method according to any one of [1] to [9], wherein, in the hematopoietic progenitor cells, the expression of KLF1 is low or the expression of FLI1 is high.
[11] The method according to [10], wherein the expression of KLF1 or FLI1 in the hematopoietic progenitor cells is correspondingly lower or higher compared to the expression in hematopoietic progenitor cells derived from KhES3.
[12] The method according to any one of [1] to [11], comprising, prior to the step (i), measuring the expression of KLF1 and/or FLI1 in the hematopoietic progenitor cells.
[13] The method according to any one of [1] to [12], wherein the step (ii) is performed for 5 days.
[14] A method for producing megakaryocytes from hematopoietic progenitor cells, comprising the following (i) and (ii) steps:
   (i) forcibly expressing c-MYC gene in hematopoietic progenitor cells and culturing the cells in a medium containing Z-DEVD-FMK, and
   (ii) arresting forced expression of the c-MYC gene in the cells obtained in the step (i) and culturing the cells.
[15] A method for producing platelets, comprising recovering platelets from the culture of the megakaryocytes obtained by the method according to any one of [1] to [14].

### Advantageous Effects of Invention

According to the present invention, it is possible to produce megakaryocytes suitable for producing platelets.

According to methods described herein, it is also possible to select hematopoietic progenitor cells suitable for producing megakaryocytes.

In inducing hematopoietic progenitor cells from stem cells and producing megakaryocytes from the hematopoietic progenitor cells by a method described, for example, in Patent Literature 3 and Patent Literature 4, the methods described herein make it possible to select appropriate hematopoietic progenitor cells, obtain megakaryocytes and mature the megakaryocytes to produce platelets. In this way, functional platelets can be produced from the stem cells without fail. The platelets thus obtained are CD42b positive and greatly contribute to clinical application.

### Brief Description of Drawings

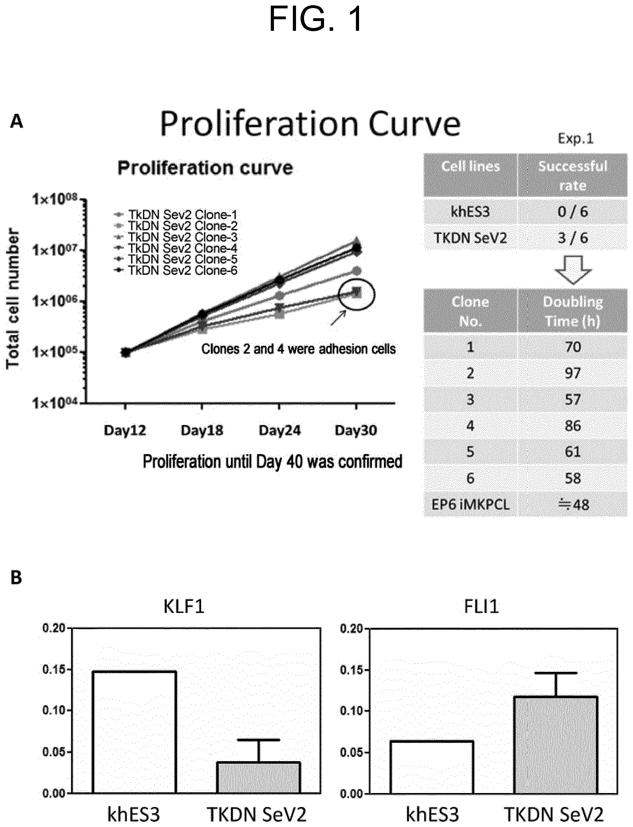

[Figure 1] Figure 1A shows proliferation curves of respective megakaryocytic cell lines (TKDN SeV2 Clone-1 to Clone-6) from Day 12 after infection with c-MYC and BCL-XL drug-responsive expression lentiviruses; and Figure 1B shows the results of expression analysis of KLF1 (left panel) or FLI1 (right panel) in hematopoietic progenitor cells derived from khES3 and TKDN SeV2.

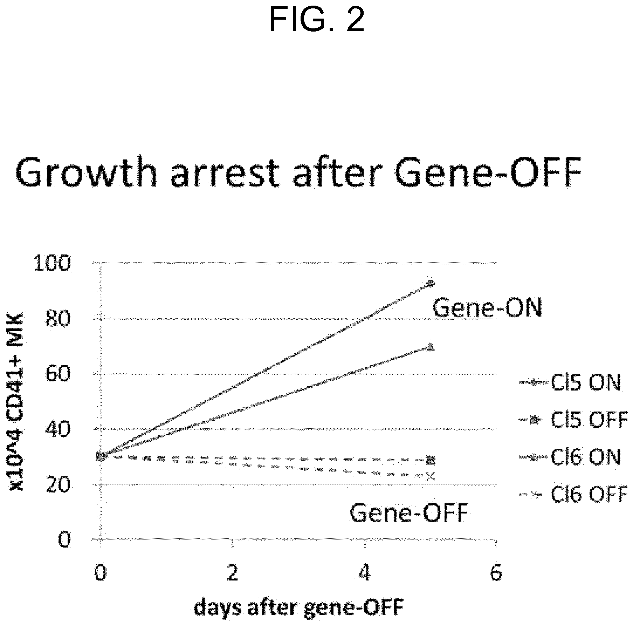

[Figure 2] Figure 2 shows proliferation curves of cells in the case of where forced expression of genes is arrested (Gene-OFF) and in the case of where the forced expression is continued (Gene-ON) in megakaryocytic cell lines (Clone-5 (Cl5) and Clone-6 (Cl6)) on Day 18 after infection with of c-MYC and BCL-XL drug-responsive expression lentivirus. In the graph, Day 0 denotes the day on which forced expression of the genes is arrested.

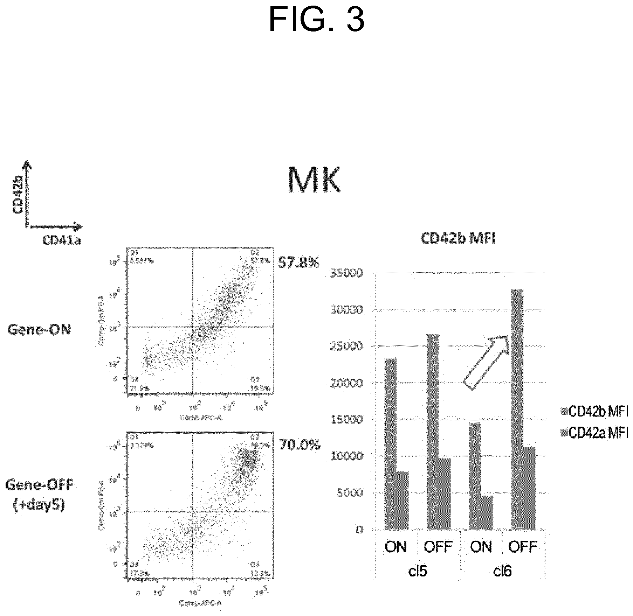

[Figure 3] Figure 3 shows the flow cytometric results of surface antigens (CD41a and CD42b) of megakaryocytes immediately after arrest of the forced expression of the genes (Gene-ON) and megakaryocytes on Day 5 after the arrest of the forced expression (Gene-OFF). The right graph shows the mean fluorescent intensity (MFI) of surface markers, CD42a (dark grey) and CD42b (light gray), in respective megakaryocytic cell lines. In the graph, the arrow points out an increase of each marker by arresting the forced expression of the genes.

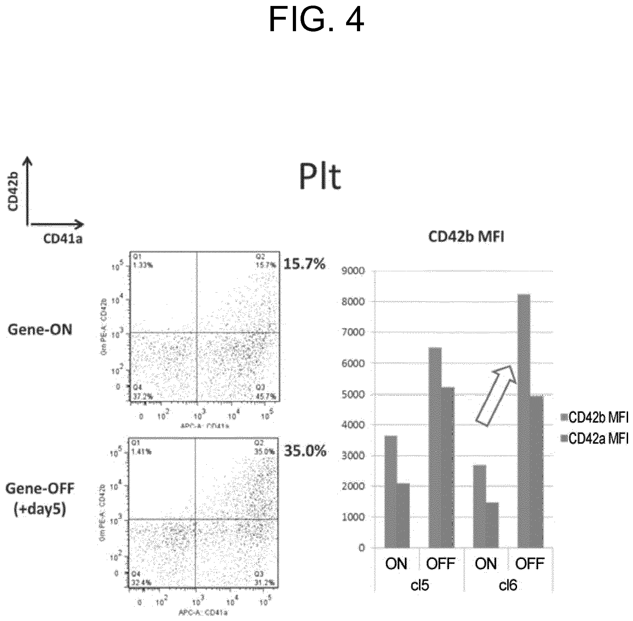

[Figure 4] Figure 4 shows the flow cytometric results of surface antigens (CD41a and CD42b) of platelets immediately after arrest of the forced expression of the genes (Gene-ON) and Day 5 after the arrest of the forced expression (Gene-OFF). The right graph shows the mean fluorescent intensity (MFI) of surface markers, CD42a (dark grey) and CD42b (light gray), in respective platelets. In the graph, the arrow points out an increase of each marker by arresting the forced expression of the genes.

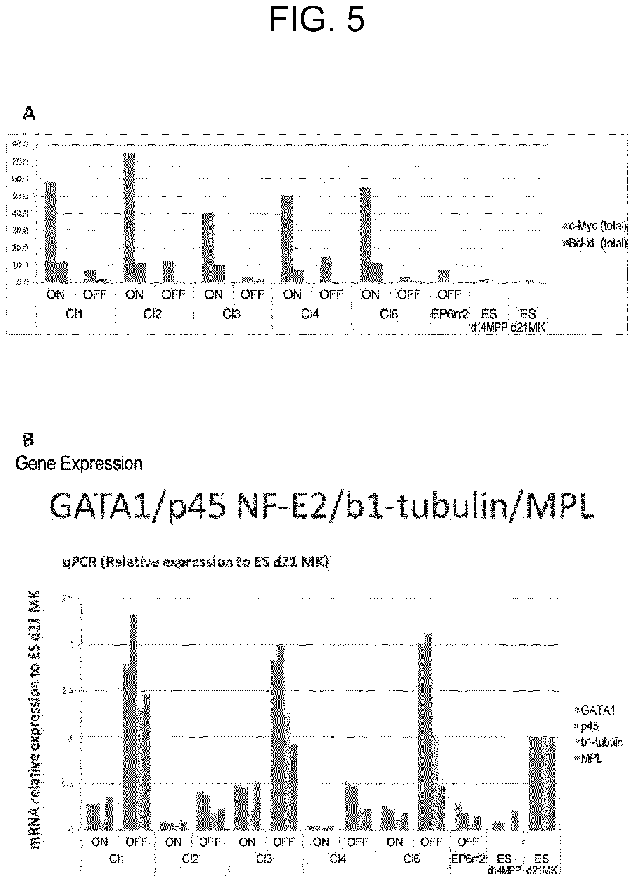

[Figure 5] Figure 5A shows expression levels of endogenous and exogenous c-Myc (light grey) and Bcl-xl (dark grey) immediately after the forced expression of the genes are arrested (ON) and on Day 3 after arrest of the gene expression (OFF), in respective iPS cell-derived megakaryocytic cell lines (Clone-1 (Cl1) to Clone-6 (Cl6)). Numerical values are relative values to ES cell-derived megakaryocytes (ES21MK). Figure 5B shows expression levels of endogenous and exogenous GATA1, p45, b1-tubulin and MPL (sequentially from the left in each condition) immediately after the expression of the genes is arrested (ON) and on Day 3 after the arrest of the gene expression (OFF), in respective iPS cell derived megakaryocytic cell lines (Clone-1 (Cl1) to Clone-6 (Cl6)). The numerical values on the vertical axis show relative values to ES cell-derived megakaryocytes (ES21MK).

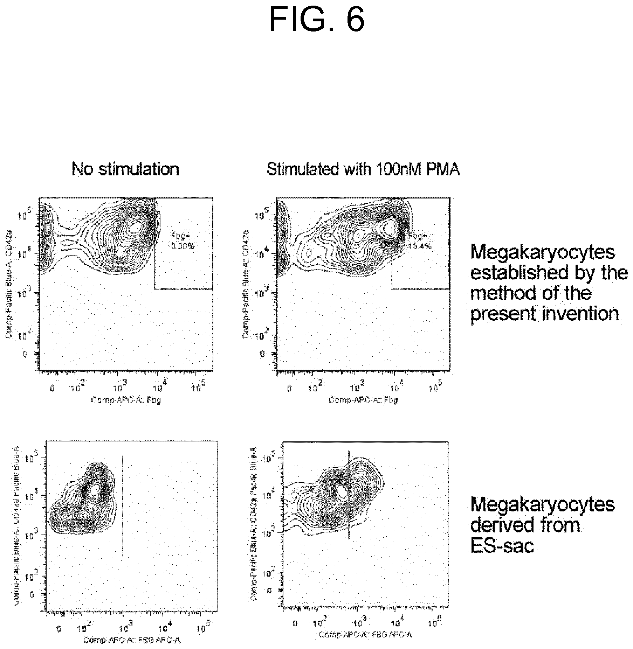

[Figure 6] Figure 6 shows the binding degree (X-axis) of APC-fibrinogen in the megakaryocytic cell line (Day 40 after culture) (upper figure) established by the method of the present invention and a control cell, i.e., megakaryocytes (Day 21) (lower figure) established by the method described in Takayama et al., Blood, 111:5298-5306, 2008, before (left) and after (right) stimulated with Phorbol 12-Myristate 13-acetate (PMA).

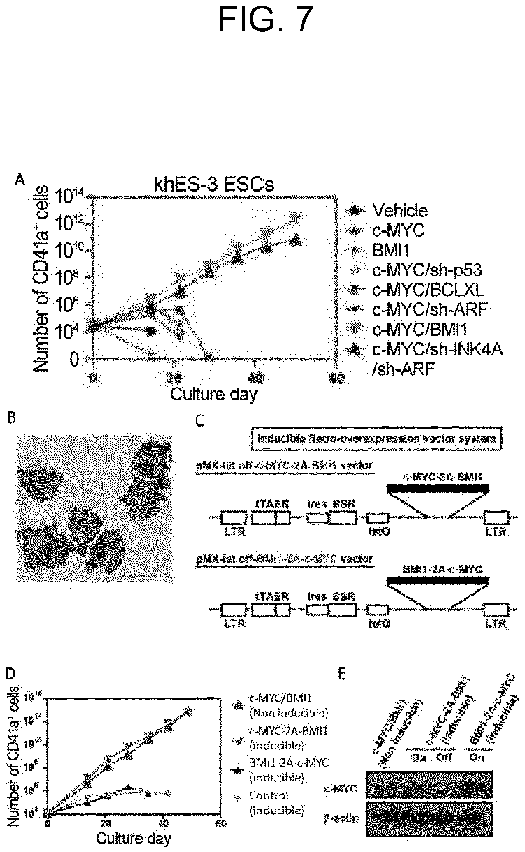

[Figure 7] Figure 7A shows the numbers of CD41a-positive cells obtained from the megakaryocytic progenitor cells produced by introducing respective genes versus culture days. Figure 7B shows an image of the megakaryocytic progenitor cells obtained by introducing c-MYC and BMI1 and stained with May-Giemsa. Figure 7C shows a schematic view of retrovirus vectors expressing c-MYC-2A-BMI1 and BMI1-2A-c-MYC. Figure 7D shows the number of CD41a-positive cells obtained from megakaryocytic progenitor cells produced by introducing respective genes versus culture days. Figure 7E shows quantitative measurement results of c-Myc protein in the case where c-MYC and BMI1 were separately introduced, in the case where c-MYC-2A-BMI1 was introduced and in the case where BMI1-2A-c-MYC was introduced.

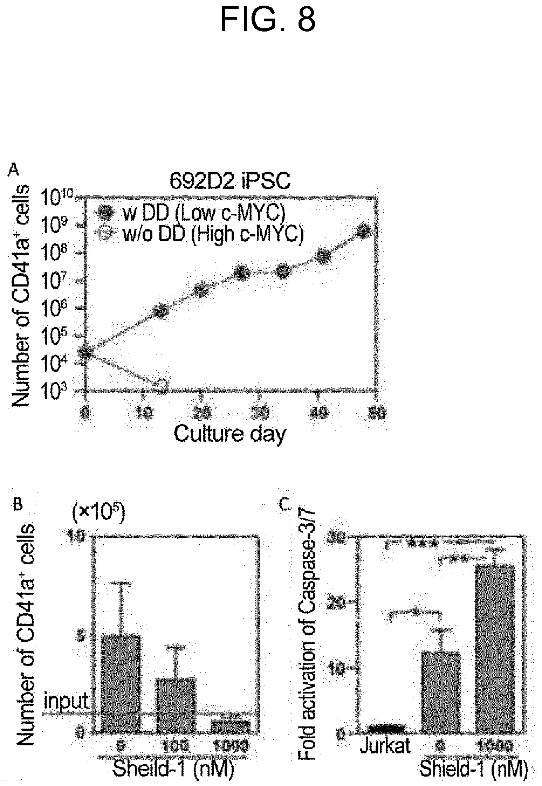

[Figure 8] Figure 8A shows the number of CD41a-positive cells obtained by introducing c-MYC-DD-2A-BMI1 (w DD) or c-MYC-2A-BMI1 (w/o DD) into hematopoietic progenitor cells. Figure 8B shows the number of CD41a-positive cells on Day 7 after c-MYC-DD-2A-BMI1-introduced cells were cultured in the mediums containing Shield-1 at different concentrations. Figure 8C shows measurement results of activity of Caspase 3/7 on Day 2 after c-MYC-DD-2A-BMI1-introduced cells were cultured in the mediums containing Shield-1 at different concentrations.

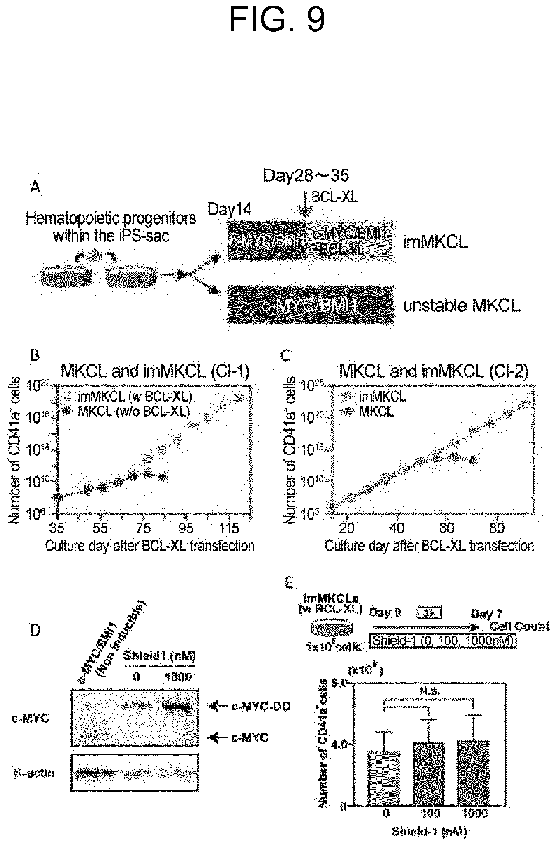

[Figure 9] Figure 9A shows a protocol for producing megakaryocytic progenitor cells by introducing c-MYC, BMI1 and BCL-XL, or c-MYC and BMI1. Figure 9B shows the results of expanding culture of megakaryocytic progenitor cell line (Cl-1) produced in accordance with the protocol shown in Figure 9A. Figure 9C shows the results of expanding culture of megakaryocytic progenitor cell line (Cl-2) produced in accordance with the protocol shown in Figure 9A. Figure 9D shows quantitative measurement result of c-Myc protein after c-MYC-DD-2A-BMI1-introduced cells were cultured in mediums containing Shield-1 at different concentrations. Figure 9E shows the number of CD41a-positive cells on Day 7 after c-MYC-DD-2A-BMI1-introduced cells were cultured in the mediums containing Shield-1 at different concentrations.

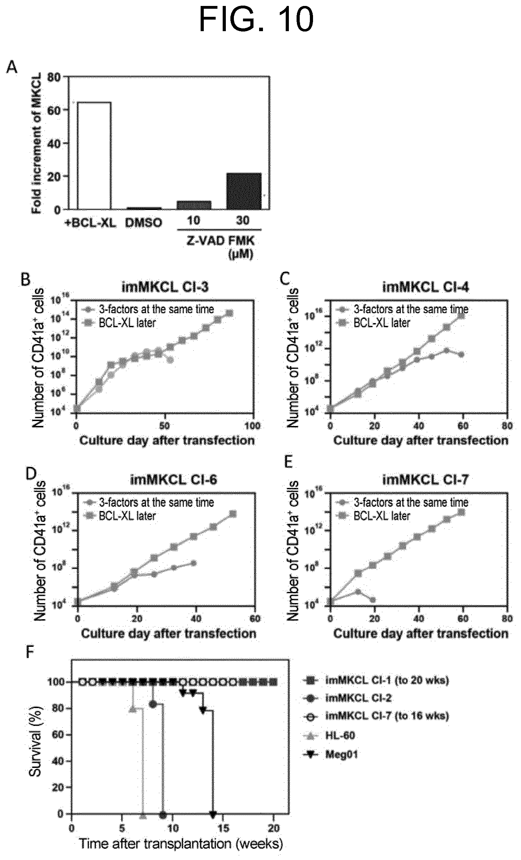

[Figure 10] Figure 10A shows the increase rate of megakaryocytic progenitor cells in the case where c-MYC-2A-BMI1 and BCL-XL were introduced or in the case where c-MYC-2A-BMI1 was introduced and cultured in a medium containing DMSO or Z-VAD-FMK. Figure 10B shows the results of expanding culture of megakaryocytic progenitor cell line (Cl-3) produced by simultaneously introducing c-MYC, BMI1 and BCL-XL. Figure 10C shows the results of expanding culture of megakaryocytic progenitor cell line (Cl-4) produced by simultaneously introducing c-MYC, BMI1 and BCL-XL. Figure 10D shows the results of expanding culture of megakaryocytic progenitor cell line (Cl-6) produced by simultaneously introducing c-MYC, BMI1 and BCL-XL. Figure 10E shows the results of expanding culture of megakaryocytic progenitor cell line (Cl-7) produced by simultaneously introducing c-MYC, BMI1 and BCL-XL. Figure 10F shows a Kaplan-Meier curve showing a survival rate of mice to which megakaryocytic progenitor cell line (Cl-1), megakaryocytic progenitor cell line (Cl-7), HL-60 (megakaryocytic cell line) or Meg01 (megakaryocytic cell line) was administered.

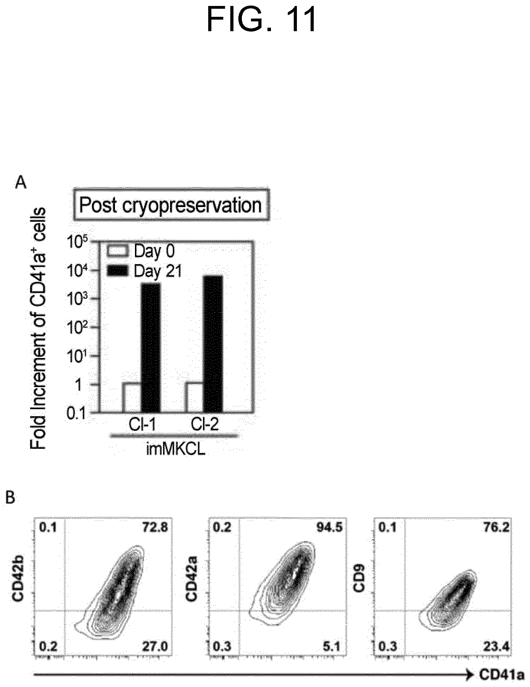

[Figure 11] Figure 11A shows the number of CD41a-positive cells on Day 0 or Day 21 after megakaryocytic progenitor cell line (Cl-1) or megakaryocytic progenitor cell line (Cl-2) cryopreserved were thawed. Figure 11B shows the flow cytometric results measured on CD41a, CD42a, CD42b and CD9 after megakaryocytic progenitor cell line (Cl-1) cryopreserved was thawed.

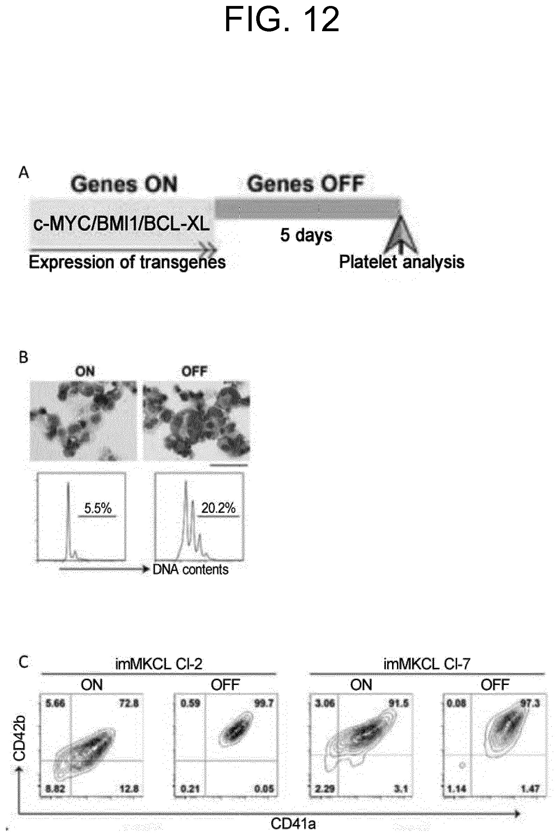

[Figure 12] Figure 12A shows a protocol for megakaryocytic progenitor cells produced by introducing c-MYC, BMI1 and BCL-XL therein into maturing megakaryocytes (to produce platelets) by arresting expression of the exogenous genes. Figure 12B shows Giemsa stained image (upper figure) of cells obtained in accordance with the protocol of Figure 12A (OFF) and cells before arrest of gene expression (ON) and the measurement results of DNA content. Figure 12C shows the flow cytometric results on CD41a and CD42b of megakaryocytic progenitor cell line (Cl-2 or Cl-7) obtained in accordance with the protocol of Figure 12A (OFF) and megakaryocytic progenitor cell line (Cl-2 or Cl-7) before arrest of gene expression (ON).

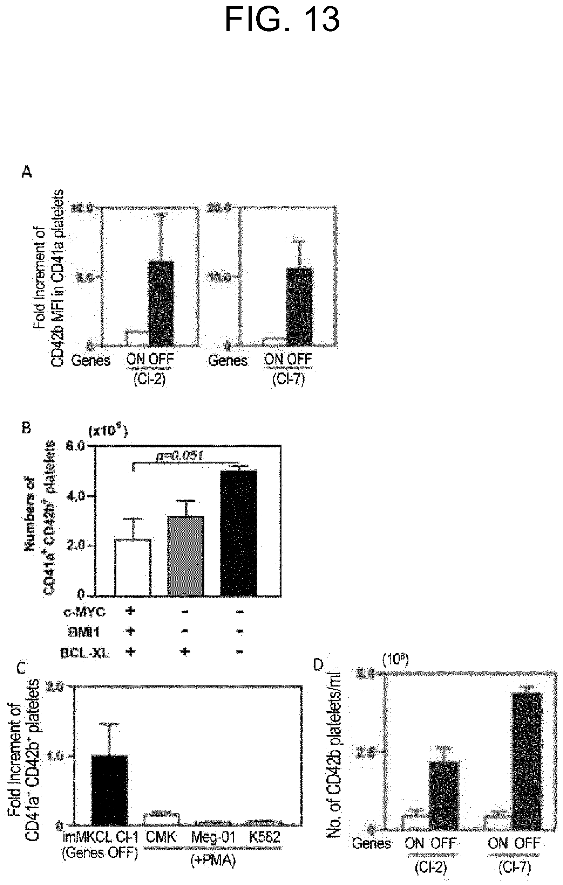

[Figure 13] Figure 13A shows an increase rate of CD41a positive/CD42b positive cells of megakaryocytic progenitor cell line (Cl-2 or Cl-7) in which expression of exogenous genes of c-MYC, BMI1 and BCL-XL was arrested (OFF) or in which expression of them was maintained (ON). Figure 13B shows the number of CD41a positive/CD42b positive cells in the cases where expression of c-MYC, BMI1 and BCL-XL was maintained, where expression of BCL-XL alone was maintained, and where expression of all genes was arrested. Figure 13C shows an increase rate of CD41a positive/CD42b positive cells in the cases where the expression of c-MYC, BMI1 and BCL-XL was maintained and where megakaryocytic cell lines (CMK, Meg-01 and K562) known in the art were stimulated with PMA. Figure 13D shows the number of CD42b positive platelets per medium (1 mL) in the case of megakaryocytic progenitor cell line (Cl-2 or Cl-7) where expression of exogenous genes, c-MYC, BMI1 and BCL-XL was arrested (OFF) or expression of them was maintained (ON).

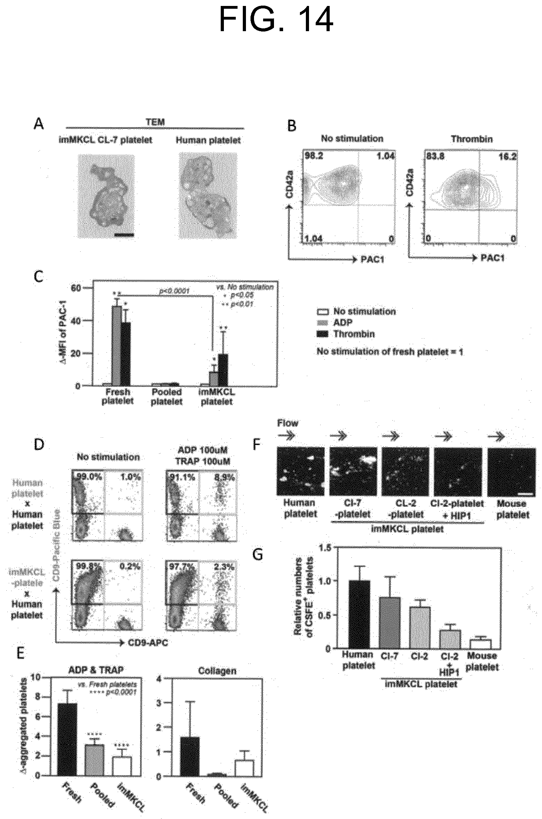

[Figure 14] Figure 14A shows a transmission electron microscopic image of platelets produced from megakaryocytic progenitor cell line (Cl-7) or platelets from blood sample. Figure 14B shows the flow cytometric results on CD42a and bound PAC-1 in the case where megakaryocytic progenitor cell line was not stimulated (No stimulation) or the case where the cell line was stimulated with thrombin (Thrombin). Figure 14C shows the binding strength of PAC-1 and platelets of a blood sample just taken (Fresh platelet), platelets pooled at 37°C for 5 days (pooled platelet) or platelets (imMKCL platelet) derived from megakaryocytic progenitor cell line, without or with stimulation by ADP or thrombin. Figure 14D shows the measurement results of agglutinated platelets (CD9-APC positive/CD9-Pacific Blue positive) in the case where Fresh platelets or imMKCL platelets were not stimulated or in the case where Fresh platelets or imMKCL platelets were stimulated with ADP and TRAP. Figure 14D shows the content rate of agglutinated platelets measured in Figure 14D (left figure) and the content rate of agglutinated platelets when stimulated with collagen (right figure). Figure 14F shows microscopic images of platelet aggregates derived from Cl-7 or Cl-2 at a flow rate of 1600S⁻¹ (direction is shown in the top part of the figures). Figure 14G shows the number of the aggregates observed in Figure 14F.

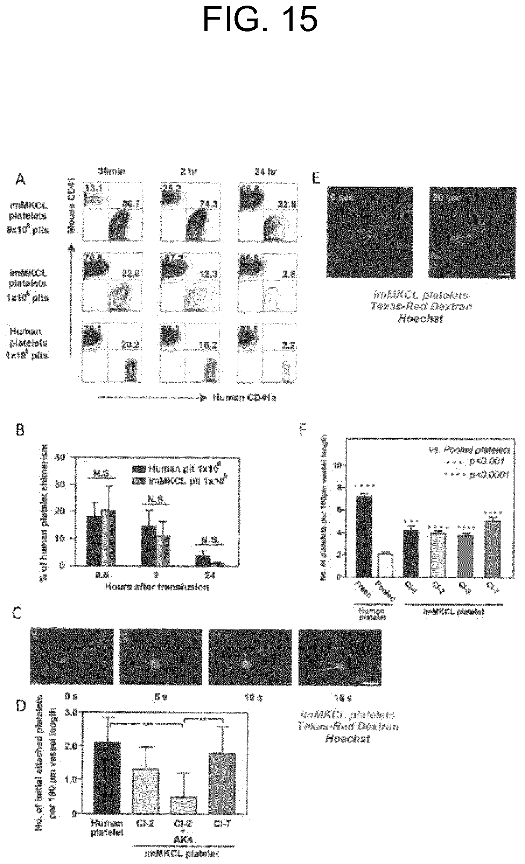

[Figure 15] Figures 15A and B show the content rate (Human CD41a positive/Mouse CD41 negative) of platelets in blood at 30 minutes, 2 hours or 24 hours after platelets (6 × 10⁸ or 1 × 10⁸) derived from megakaryocytic progenitor cell line or platelets (1 × 10⁸) derived from a blood sample were administered to a mouse. Figure 15C shows confocal microscopic images of *in vivo* behavior of platelets (green) derived from megakaryocytic progenitor cell line in a blood vessel (red) taken over time. Figure 15D shows the number of platelets derived from megakaryocytic progenitor cell line adhered per blood vessel (100 µm). Figure 15E shows microscopic images of platelets (green) derived from megakaryocytic progenitor cell line in thrombus (20 sec) generated in a portion damaged with laser irradiation. Figure 15E shows the number of platelets contained in thrombus in the cases where platelets (Fresh) in a blood sample, pooled platelets (pooled) and platelets derived from megakaryocytic progenitor cell lines (Cl-1, Cl-2, Cl-3 and Cl-7) were administered to a mouse.

### Description of Embodiments

### (Production method of megakaryocytes)

The present invention provides a method for producing megakaryocytes from hematopoietic progenitor cells.

An aspect of the method for producing a megakaryocyte according to the present invention includes a step of forcibly expressing an apoptosis suppression gene and an oncogene in hematopoietic progenitor cells and culturing the cells and a step of arresting the forced expression of the apoptosis suppression gene and oncogene and culturing the cells. In the present invention, the cells obtained in the step of forcibly expressing an apoptosis suppression gene and oncogene in hematopoietic progenitor cells and culturing the cells may be regarded as megakaryocytic progenitor cells.

The "megakaryocytes" in the present invention may be multinucleated cells, which, for example, include cells characterized as being CD41a positive/CD42a positive/CD42b positive. Other than this, cells characterized by expressing GATA1, FOG1, NF-E2 and β1-tubulin therein may be included. The megakaryocyte multinucleated refers to a cell or a cell population in which the number of nuclei has relatively increased than hematopoietic progenitor cells. For example, provided that the number of nuclei of hematopoietic progenitor cells, to which the method of the present invention is to be applied, is 2N, cells having nuclei of 4N or more is defined as multinucleated megakaryocytes. In the present invention, the megakaryocytes may be immortalized as a megakaryocytic cell line or a cloned cell population.

The "megakaryocytic progenitor cell" used in the present invention is defined as a cell, which is to be matured into a megakaryocyte and not multinucleated. Examples of the megakaryocytic progenitor cells include cells characterized by CD41a positive/CD42a positive/CD42b slightly positive. The megakaryocytic progenitor cells of the present invention are preferably cells which can be proliferated by expanding culture, more specifically, cells which can be proliferated by expanding culture in appropriate conditions for at least 60 days. In the present invention, the megakaryocytic progenitor cell may or may not be cloned. Although it is not particularly limited, a cloned megakaryocytic progenitor cells may be sometimes called as megakaryocytic progenitor cell line.

In the present invention, the hematopoietic progenitor cell refers to a cell that can be differentiated into blood cells such as lymphocytes, acidocytes, neutrophils, basophils, erythrocytes and megakaryocytes. In the present invention, a hematopoietic progenitor cell and a hematopoietic stem cell are indistinguishably used and regarded as the same cell unless otherwise specified. The hematopoietic stem cell and hematopoietic progenitor cell can be identified by, for example, surface antigens, CD34 and/or CD43 being positive. In the present invention, the hematopoietic stem cells may be hematopoietic progenitor cells differentiation-induced from pluripotent stem cells, hematopoietic stem cells and progenitor cells derived from umbilical cord blood, bone-marrow blood, and peripheral blood. For example, the hematopoietic stem cells can be prepared from a net-like construct (referred to also as ES-sac or iPS-sac), which is obtained by culturing pluripotent stem cells on C3H10T1/2 in the presence of VEGF in accordance with the method described in Takayama N., et al. J Exp Med. 2817-2830 (2010). Here, the "net like construct" refers to a three-dimensional cystic construct (having an interior space) derived from pluripotent stem cella, formed of e.g., endothelial cells and containing hematopoietic progenitor cells. Other than this, as a method of producing hematopoietic progenitor cells from pluripotent stem cells, for example, a method by forming an embryoid body and addition of a cytokine (Chadwick et al. Blood 2003, 102: 906-15, Vijayaragavan et al. Cell Stem Cell 2009, 4: 248-62, Saeki et al. Stem Cells 2009, 27: 59-67) or a co-culture method with stroma cells derived from xenogeneic species (Niwa A et al. J Cell Physiol. 2009 Nov; 221 (2) : 367-77.) is mentioned.

In the present invention, preferable hematopoietic progenitor cells are hematopoietic progenitor cells in which expression of KLF1 gene is low or expression of FLI1 gene is high. Accordingly, in producing megakaryocytes, a step of selecting hematopoietic progenitor cells where expression of KLF1 is low or expression of FLI1 is high may be included. Here, the "expression of KLF1 is low" means that expression of KLF1 is lower than that of a control. The control is not particularly limited and can be appropriately selected by those skilled in the art based on e.g., literatures or experience. For example, hematopoietic progenitor cells produced from khES3 in accordance with the method described in Takayama N., et al. J Exp Med. 2817-2830 (2010) may be used. KLF1 refers to a gene described in NCBI, Accession Number NM_006563.

The "expression of FLI1 is low" means that expression of FLI1 is lower compared to a control. The control is not particularly limited and can be appropriately selected by those skilled in the art based on e.g., literatures or experience. For example, hematopoietic progenitor cells produced from khES3 in accordance with the method described in Takayama N., et al. J Exp Med. 2817-2830 (2010) may be used. FLI1 refers to a gene described in NCBI, Accession Numbers NM_001167681, NM_001271010, NM_001271012 or NM_002017.

Expression of a gene can be measured in accordance with a method known to those skilled in the art, such as a DNA chip method, Southern blot method, Northern blot method or RT-PCR (Polymerase Chain Reaction) method.

In the step of selecting hematopoietic progenitor cells in which expression of KLF1 is low or expression of FLI1 is high, hematopoietic progenitor cells produced from khES3 cells as mentioned above or hematopoietic progenitor cells in a living body may be used as a control. Alternatively, two or more hematopoietic progenitor cells are selected and compared, and then a hematopoietic progenitor cell(s) having lower KLF1 expression or higher FLI1 expression may be selected. Other than this, when hematopoietic progenitor cells produced from pluripotent stem cells are used, among hematopoietic progenitor cells simultaneously produced, hematopoietic progenitor cells having lower KLF1 expression or higher FLI1 expression may be selected, and used in the present invention.

In the specification, the case "expression is low or high" is not limited to the case where expression is significantly low or high compared to a control and may include the case where those skilled in the art can recognize that expression tends to be low or high.

In the present invention, the pluripotent stem cells refers to stem cells having a pluripotency, i.e., an ability to differentiate into any types of cells present in a living body, and simultaneously having a proliferation potency. Examples of the pluripotent stem cells include embryonic stem (ES) cells (wherein human embryonic stem cells obtained by means of a process in which human embryos are destroyed are excluded), non-human embryonic stem cells obtained by nuclear transplantation from cloned embryo (ntES cells), non-human sperm stem cells (GS cells), non-human embryonic germ cells (EG cells), induced pluripotent stem (iPS) cells, pluripotent cells derived from cultured fibroblast cells or bone-marrow stem cells (Muse cells) and Stimulus-Triggered Acquisition of Pluripotency cells (STAP cells).

### (A) Embryonic stem cells

The ES cells are stem cells established from an inner cell mass of a nascent embryo (for example, blastocyst) of a mammal such as a mouse and having a pluripotency and proliferation potency based on self-replication.

The ES cells are embryonic stem cells derived from an inner cell mass of a blastocyst, which is an 8-cell period of a fertilized egg, i.e., an embryo after a morula, having an ability to differentiate into any types of cells constituting an adult body (called pluripotency) and proliferation potency based on self-replication. The ES cells were found in a mouse in 1981 (M. J. Evans and M. H. Kaufman (1981), Nature 292: 154-156) and thereafter, an ES cell line was established in a primate such as a human and a monkey (J. A. Thomson et al. (1998), Science 282: 1145-1147; J.A. Thomson et al. (1995), Proc. Natl. Acad. Sci. USA, 92: 7844-7848; J. A. Thomson et al. (1996), Biol. Reprod., 55: 254-259; J.A. Thomson and V. S. Marshall (1998), Curr. Top. Dev. Biol., 38: 133-165).

The ES cells can be established by taking out an inner cell mass from a blastocyst of a fertilized egg of a subject animal and culturing the inner cell mass on fibroblast feeder cells. Furthermore, cells can be maintained by passage culture using a culture solution supplemented with a substance such as a leukemia inhibitory factor (LIF) and a basic fibroblast growth factor (bFGF). A method for establishing and maintaining ES cells of a human and a monkey is described, for example, in USP 5,843,780; Thomson JA, et al. (1995), Proc Natl. Acad. Sci. U S A. 92: 7844-7848; Thomson JA, et al. (1998), Science. 282: 1145-1147; H. Suemori et al. (2006), Biochem. Biophys. Res. Commun., 345: 926-932; M. Ueno et al. (2006), Proc. Natl. Acad. Sci. USA, 103: 9554-9559; H. Suemori et al. (2001), Dev. Dyn., 222: 273-279; H. Kawasaki et al. (2002), Proc. Natl. Acad. Sci. USA, 99: 1580-1585; and Klimanskaya I, et al. (2006), Nature. 444: 481-485.

As a culture solution for preparing ES cells, for example, DMEM/F-12 culture solution supplemented with 0.1 mM 2-mercaptoethanol, 0.1 mM non-essential amino acids, 2 mM L-glutamic acid, 20% KSR and 4 ng/mL bFGF, is used. Using the culture solution, the human ES cells can be maintained at 37°C under a 5% CO₂ moist atmosphere (H. Suemori et al. (2006), Biochem. Biophys. Res. Commun., 345: 926-932). The ES cells must be subcultured every 3 to 4 days. At this time, the subculture can be performed, for example, by use of 0.25% trypsin and 0.1 mg/mL collagenase IV in PBS containing 1 mM CaCl₂ and 20% KSR.

The ES cells can be selected by Real-Time PCR method, generally, based on expression of gene markers such as alkaline phosphatase, Oct-3/4 and Nanog used as indices. Particularly, in selecting human ES cells, expression of gene markers such as OCT-3/4, NANOG and ECAD can be used as indices (E. Kroon et al. (2008), Nat. Biotechnol., 26: 443-452).

Human ES cell lines which are described herein as reference examples, more specifically, WA01 (H1) and WA09 (H9), can be obtained from the WiCell Reserch Institute; whereas KhES-1, KhES-2 and KhES-3 can be obtained from the Institute for Frontier Medical Sciences Kyoto University (Kyoto, Japan).

### (B) Sperm stem cells

The sperm stem cells, which are pluripotent stem cells derived from the testis, is cells of origin for spermatogenesis. The sperm stem cells, as are the same as in ES cells, can be differentiation-induced into various lines of cells. If the sperm stem cells are transplanted into, for example, a mouse blastocyst, a chimera mouse can be created (M. Kanatsu-Shinohara et al. (2003) Biol. Reprod., 69: 612-616; K. Shinohara et al. (2004), Cell, 119: 1001-1012). The sperm stem cells can be self-replicated in a culture solution containing a glial cell line-derived neurotrophic factor (GDNF) and produced by repeating subculture in the same culture condition as in producing ES cells (Masanori Takebayashi et al. (2008), Experimental Medicine, vol. 26, No. 5 (extra number), pages 41 to 46, Yodosha (Tokyo, Japan)).

### (c) Non-human embryonic germ cells

The embryonic germ cells are cells established from primordial germ cells in an embryonic stage and having the same pluripotency as in ES cells. The embryonic germ cells can be established by culturing primordial germ cells in the presence of substances such as LIF, bFGF, and a stem cell factor (Y. Matsui et al. (1992), Cell, 70: 841-847; J.L. Resnick et al. (1992), Nature, 359: 550-551).

### (D) Induced pluripotent stem cell

The induced pluripotent stem (iPS) cells, which can be prepared by introducing predetermined reprogramming factors in the form of DNA or a protein into somatic cells, are artificial stem cells derived from somatic cells having almost the same properties as those of ES cells, such as pluripotency and proliferation potency based on self-replication (K. Takahashi and S. Yamanaka (2006) Cell, 126: 663-676; K. Takahashi et al. (2007), Cell, 131: 861-872; J. Yu et al. (2007), Science, 318: 1917-1920; Nakagawa, M.et al., Nat. Biotechnol. 26: 101-106 (2008); International Publication No. WO 2007/069666). The reprogramming factors may be constituted of genes specifically expressed in the ES cells or products or non-coding RNAs of such genes; or genes playing important roles in maintaining undifferentiated state of ES cells or products or non-coding RNAs of such genes; or small molecule compounds. Examples of genes used as the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, Tbx3 or Glis1. These reprogramming factors may be used singly or in combination. Example of combinations of reprogramming factors includes those described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26: 2467-2474, Huangfu D, et al. (2008), Nat Biotechnol. 26: 1269-1275, Shi Y, et al. (2008), Cell Stem Cell, 3, 568-574, Zhao Y, et al. (2008), Cell Stem Cell, 3: 475-479, Marson A, (2008), Cell Stem Cell, 3, 132-135, Feng B, et al. (2009), Nat Cell Biol. 11: 197-203, R.L. Judson et al., (2009), Nat. Biotech., 27: 459-461, Lyssiotis CA, et al. (2009), Proc Natl Acad Sci U S A. 106: 8912-8917, Kim JB, et al. (2009), Nature. 461: 649-643, Ichida JK, et al. (2009), Cell Stem Cell. 5: 491-503, Heng JC, et al. (2010), Cell Stem Cell. 6: 167-74, Han J, et al. (2010), Nature. 463: 1096-100, Mali P, et al. (2010), Stem Cells. 28: 713-720 and Maekawa M, et al. (2011), Nature. 474: 225-9.

Examples of the above reprogramming factors may include factors to be used for enhancing efficiency of establishment such as
a histone deacetylase (HDAC) inhibitor (for example, a small molecule inhibitor such as valproic acid (VPA), trichostatin A, sodium butyrate, MC 1293 and M344; and a nucleic acid expression inhibitor such as siRNA and shRNA against HDAC (e.g. HDAC1 siRNA Smartpool·(Millipore), HuSH 29mer shRNA Constructs against HDAC1 (OriGene));
an MEK inhibitor (for example, PD184352, PD98059, U0126, SL327 and PD0325901);
a Glycogen synthase kinase-3 inhibitor (for example, Bio and CHIR99021);
a DNA methyltransferase inhibitor (e.g., 5-azacytidine);
a histone methyl transferase inhibitor (for example, a small molecule inhibitor such as BIX-01294; and a nucleic acid expression inhibitor such as siRNA and shRNA against Suv39hl, Suv39h2, SetDBl and G9a);
an L-channel calcium agonist (for example, Bayk8644);
butyric acid, TGFβ inhibitor or ALK5 inhibitor (for example, LY364947, SB431542, 616453 and A-83-01);
a p53 inhibitor (for example siRNA and shRNA against p53), an ARID3A inhibitor (for example, siRNA and shRNA against ARID3A);
an miRNA such as miR-291-3p, miR-294, miR-295 and mir-302;
Wnt Signaling (for example, soluble Wnt3a);
Neuropeptide Y;
prostaglandins (for example, prostaglandin E2 and prostaglandin J2);
hTERT; SV40LT; UTF1; IRX6; GLISl; PITX2; and DMRTBl.

In the present specification, these factors for use in improving efficiency of establishment may be indistinguishably used as the reprogramming factor.

The reprogramming factor in the form of a protein may be introduced into a somatic cell, for example, by means of lipofection, fusion with a cell membrane permeable peptide (for example, HIV-derived TAT and polyarginine) and microinjection.

In contrast, the reprogramming factor in the form of DNA may be introduced into a somatic cell, for example, by means of a vector such as a virus, a plasmid and an artificial chromosome; lipofection; liposome; and microinjection. Example of the virus vector includes a retrovirus vector, a lentivirus vector (see Cell, 126, pp. 663-676, 2006; Cell, 131, pp. 861-872, 2007; Science, 318, pp.1917-1920, 2007), an adenovirus vector (Science, 322, 945-949, 2008), adeno-associated virus vector and a Sendai virus vector (WO 2010/008054). Example of the artificial chromosomal vector includes a human artificial chromosome (HAC), a yeast artificial chromosome (YAC) and a bacterial artificial chromosome (BAC, PAC). As the plasmid, a plasmid for a mammal cell (Science, 322: 949-953, 2008) can be used. The vector may include control sequences such as a promoter, an enhancer, a ribosome-binding sequence, a terminator and a polyadenylation site in order to express an nuclear-reprogramming substance; and if necessary, further include, a selection marker sequence such as a drug resistance gene (for example a kanamycin-resistant gene, an ampicillin-resistant gene, a puromycin-resistant gene), a thymidine kinase gene and a diphtheria toxin gene; and a reporter gene sequence such as green fluorescent protein (GFP), β glucuronidase (GUS) and FLAG. In the vector, LoxP sequences may be arranged upstream and downstream of a gene encoding a reprogramming factor or a promoter and a gene encoding a reprogramming factor linked thereto to excise it (them) after the vector is introduced into a somatic cell.

The reprogramming factor in the form of RNA may be introduced into a somatic cell, for example, by means of e.g., lipofection and microinjection. To suppress decomposition, 5-methylcytidine and pseudouridine (TriLink Biotechnologies)-incorporated RNA may be used (Warren L, (2010) Cell Stem Cell. 7: 618-630).

Example of a culture solution for inducing an iPS cell includes 10 to 15% FBS-containing DMEM, DMEM/F12 or DME culture solution (these culture solutions may appropriately contain e.g., LIF, penicillin/streptomycin, puromycin, L-glutamine, nonessential amino acids and β-mercaptoethanol) or a commercially available culture solution (for example, a culture solution for culturing a mouse ES cell (TX-WES culture solution, Thrombo X), a culture solution for culturing a primate ES cell (a culture solution for primate ES/iPS cells, Reprocell Inc.) and a non-serum medium (mTeSR, Stemcell Technologies)].

The culture is, for example, performed by the following method. Somatic cells are brought into contact with reprogramming factors at 37°C, 5% CO₂, in 10% FBS-containing DMEM or DMEM/F12 culture solution, and cultured for about 4 to 7 days, reseeded on feeder cells (for example, mitomycin C treated STO cells or SNL cells) and started culturing in a bFGF-containing culture solution for primate ES cells from about Day 10 after the contact with the reprogramming factors. IPS cell-like colonies can be obtained in about 30 to 45 days or more from the contact.

Alternatively, the somatic cells are cultured on feeder cells (for example, mitomycin C treated STO cells or SNL cells) at 37°C, 5% CO₂, in 10% FBS-containing DMEM culture solution (which may further appropriately contain e.g., LIF, penicillin/streptomycin, puromycin, L-glutamine, nonessential amino acids and β-mercaptoethanol). In this case, ES-like colonies can be obtained in about 25 to 30 days or more. Desirably, a method using the somatic cells to be reprogrammed in place of a feeder cell (Takahashi K, et al. (2009), PLoS One. 4: e8067 or WO2010/137746), and a method using an extracellular substrate (for example, Laminin-5 (WO2009/123349) or matrigel (company: BD)) in place of a feeder cell are taken as examples.

Other than this, a culture method using a serum-free medium is taken as an example (Sun N, et al. (2009), Proc Natl Acad Sci U S A. 106: 15720-15725). To improve efficiency of establishment, iPS cells may be established in a low oxygen condition (oxygen concentration of 0.1% or more and 15% or less) (Yoshida Y, et al. (2009), Cell Stem Cell 5: 237-241 or WO2010/013845).

During the culturing, the culture solution is exchanged with a fresh culture solution once a day from Day 2 after initiation of the culturing. The number of somatic cells to be used for nuclear reprogramming is not limited; however, the number falls within the range of about 5 × 10³ to about 5 × 10⁶ cells per culture dish (100 cm²).

IPS cells can be selected based on the shape of colony formed. In the case where a drug resistance gene to be expressed in conjunction with expression of genes (for example, Oct3/4 or Nanog), which are expressed when the somatic cells are reprogrammed, is introduced as a marker gene, the established iPS cells can be selected by performing culture in a culture solution (selective culture solution) containing the corresponding drug. If the marker gene is a fluorescent protein gene, iPS cells can be selected by a fluorescent microscope; if the marker gene is a luminescent enzyme gene, by adding a luminescent substrate; whereas if the marker gene is a color-emitting enzyme gene, by adding a chromogenic substrate.

The term "somatic cell" used in the present specification refers to all animal cells (preferably, mammal cells including human cells) except germ-line cells or totipotent cells, such as ovum, oocytes and ES cells. Example of the somatic cells includes, but not limited to, fetal somatic cells, newborn infant somatic cells and matured healthy or sick somatic cells. In addition, primary cultured cells, subcultured cells and established cell lines are included in the somatic cells. Specific examples of the somatic cells include
(1) tissue stem cells (somatic stem cells) such as nerve stem cells, hematopoietic stem cells, mesenchymal stem cells and pulpal stem cells; (2) tissue precursor cells; and (3) differentiated cells such as lymphocytes, epithelial cells, endothelial cells, muscle cells, fibroblasts (skin cells), hair cells, hepatic cells, gastric mucosal cells, intestinal cells, splenic cells, pancreatic cells (pancreatic exocrine cells), brain cells, pulmonary cells, kidney cells and adipose cells.

In the present invention, in consideration that iPS cell-derived platelets are used as a transplantation material, it is desirable that the iPS cell-derived platelets hardly cause a rejection. In view of this, it is desirable to use somatic cells whose HLA gene type is identical or substantially identical with that of a transplant-recipient, for producing iPS cells. Here, "substantially the same" means that the HLA gene type is similar to such an extent that an immune reaction to transplanted cells can be suppressed by an immune suppressive agent. To be more specific, for example, a somatic cell having an HLA type gene identical in three gene loci (HLA-A, HLA-B and HLA-DR) or in four gene loci (HLA-A, HLA-B, HLA-DR and HLA-C) to those of a somatic cell of the recipient may be used.

### (E) Non-human ES cells derived from cloned embryo obtained by nuclear transplantation

Nuclear transfer-derived ES cells (nt ES cell) are ES cells derived from cloned embryos prepared by nuclear transplantation technique and having almost the same characteristics as in fertilized egg-derived ES cells (T. Wakayama et al. (2001), Science, 292: 740-743; S. Wakayama et al. (2005), Biol. Reprod., 72: 932-936; J. Byrne et al. (2007), Nature, 450: 497-502). More specifically, ES cells established from an inner cell mass of a blastocyst derived from a cloned embryo, which is obtained by replacing the nucleus of an unfertilized egg with the nucleus of a somatic cell, are the nt ES (nuclear transfer-derived ES) cells. In preparing the nt ES cells, a nuclear transplantation technique (J. B. Cibelli et al. (1998), Nature Biotechnol., 16: 642-646) and ES cell preparation technique are used in combination (Kiyoka Wakayama et al. (2008), Experimental Medicine, vol. 26, No. 5 (extra number), pages 47 to 52). In the nuclear transplantation, reprogramming can be made by injecting the nucleus of a somatic cell into a mammalian denucleated unfertilized egg and culturing the egg for several hours.

### (F) Multilineage-differentiating Stress Enduring cells (Muse cells)

The Muse cells are pluripotent stem cells produced by the method described in WO2011/007900, and more specifically, cells having a pluripotency, which is obtained by treating fibroblast cells or bone-marrow stromal cells with trypsin for hours, preferably 8 hours or 16 hours, and subjecting the treated cells to suspension culture. The Muse cells are positive for SSEA-3 and CD105.

### (G) Stimulus-Triggered Acquisition of Pluripotency cells (STAP cells)

The STAP cells are pluripotent stem cells produced by the method described in WO2013/163296, and for example, obtained by culturing somatic cells in an acidic solution of pH5.4 to 5.8 for 30 minutes. The STAP cells are positive for SSEA-4 and E-cadherin.

In the present invention, preferable pluripotent stem cells are those capable of producing hematopoietic progenitor cells which can be induced differentiation into megakaryocytes. The selection of such pluripotent stem cells can be made based on whether or not hematopoietic progenitor cells in which expression of KLF1 is lower or expression of FLI1 is higher are obtained. As the method for producing hematopoietic progenitor cells from pluripotent stem cells and the method for measuring the expression of KLF1 and FLI1 herein, the aforementioned methods can be employed.

Also described herein is a method for selecting pluripotent stem cells suitable for producing megakaryocytes, including a step (1) of producing hematopoietic stem cells from pluripotent stem cells and step (2) of measuring expression of KLF1 and expression of FLI1 in the hematopoietic progenitor cells produced in the step (1).

In the present application, the term "oncogene" refers to a gene whose expression, structure or function differs from a normal gene, and thereby causing canceration of a normal cell. Example of oncogenes includes an MYC family gene, a Src family gene, a Ras family gene, a Raf family gene and a protein kinase family gene such as c-Kit, PDGFR and Abl. Example of the MYC family gene includes c-MYC, N-MYC and L-MYC. In the presently claimed invention, the oncogene is a c-MYC gene. The c-MYC gene is a gene having a nucleotide sequence represented, for example, by NCBI Accession Number NM_002467. Furthermore, the c-MYC gene may include a homologue of the gene. The c-MYC gene homologue is a gene having a cDNA sequence consisting of substantially the same nucleotide sequence as that, for example, represented by NCBI Accession Number NM_002467. The cDNA consisting of substantially the same nucleotide sequence as that represented by NCBI Accession Number NM_002467 refers to DNA consisting of a sequence having an identity of about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% with the sequence represented by NCBI Accession Number NM_002467 or DNA capable of hybridizing with DNA consisting of a complementary sequence to the nucleotide sequence represented by NCBI Accession Number NM_002467 in a stringent condition. The proteins encoded by these DNA molecules are specified to contribute to proliferation of cells in any differentiation stage, such as hematopoietic progenitor cells.

The stringent condition herein refers to a hybridization condition easily determined by those skilled in the art and generally refers to an experiment condition empirically determined depending upon the probe length, washing temperature and a salt concentration. Generally, as the length of a probe increases, the temperature for appropriate annealing increases; conversely, as the length of a probe decreases, the temperature decreases. Hybrid formation generally varies depending upon the ability of a complementary chain to reanneal at a temperature slightly lower than its melting point.

For example, as a low stringent condition, as the condition during the filter-washing step after hybridization, the condition of 37°C to 42°C, 0.1 × SSC and 0.1% SDS solution may be taken. In contrast, as a high stringent condition, for example, as the condition during the washing step, the condition of 65°C, 5 × SSC and 0.1% SDS may be taken. If the stringent condition becomes more severe, a highly homologous polynucleotide can be obtained.

In the present invention, it is preferable to suppress the expression level of c-MYC. Thus, c-MYC may encode a protein fused with a destabilized domain. The destabilized domain may be purchased from ProteoTuner or Clontech and put in use.

In the present application, the "apoptosis suppression gene" is not particularly limited as long as it suppresses apoptosis. Examples of apoptosis suppression genes include BCL2 gene, BCL-XL gene, Survivin and MCL1. In the presently claimed invention, the apoptosis suppression gene is BCL-XL. The BCL-XL gene refers to a gene consisting of, for example, a nucleotide sequence represented by NCBI Accession Number NM_001191 or NM_138578. Furthermore, the BCL-XL gene may include a homologue thereof. The BCL-XL gene homologue is a gene having a cDNA sequence consisting of substantially the same nucleotide sequence as that, for example, represented by NCBI Accession Number NM_001191 or NM_138578. The cDNA consisting of substantially the same nucleotide sequence as that represented by NCBI Accession Number NM_001191 or NM_138578 refers to DNA consisting of a sequence having an identity of about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98% and most preferably about 99% with the sequence represented by NCBI, Accession Number NM_001191 or NM_138578 or DNA capable of hybridizing with DNA consisting of a complementary sequence to the nucleotide sequence represented by NCBI Accession Number NM_001191 or NM_138578 in a stringent condition. The protein encoded by the DNA molecule is specified to have an effect of suppressing apoptosis.

In methods described herein, a product obtained by a step of forcibly expressing any one of the following (i) to (iii) genes in hematopoietic progenitor cells and proliferating the hematopoietic progenitor cells by culturing the cells can be used:
(i) a gene that suppresses the expression of p16 gene or p19 gene;
(ii) a gene that suppresses the expression of Ink4a/Arf gene; and
(iii) a polycomb gene.

As the (i) to (iii) genes, for example, BMI1, Mel18, Ringla/b, Phc1/2/3, Cbx2/4/6/7/8, Ezh2, Eed, Suz12, HDAC, and Dnmt1/3a/3b can be used. Of them, BMI1 gene is particularly preferable. The BMI1 gene is, for example, a gene consisting of a nucleotide sequence represented by NCBI Accession Number NM_005180. The BMI1 gene may include a homologue thereof. The BMI1 gene homologue is a gene having a cDNA sequence consisting of substantially the same nucleotide sequence as that, for example, represented by NCBI Accession Number NM_005180. The cDNA consisting of substantially the same nucleotide sequence as that represented by NCBI Accession Number NM_005180 refers to DNA constituted of a sequence having a homology of about 60% or more, preferably about 70% or more, more preferably about 80%, 81%, 82%, 83%, 84%, 85%, 86%, 87%, 88%, 89%, 90%, 91%, 92%, 93%, 94%, 95%, 96%, 97%, 98%, and most preferably about 99% with the sequence represented by NCBI, Accession Number NM_005180 or DNA capable of hybridizing with DNA consisting of a complementary sequence to the nucleotide sequence represented by NCBI, Accession Number NM_005180 in a stringent condition. The protein encoded by the DNA molecule is specified to suppress an oncogene-induced cellular senescence in the cells where an oncogene such as an MYC family gene is expressed and accelerate proliferation of the cells.

In the present invention, when a gene selected from the group consisting of (i) a gene that suppresses the expression of p16 gene or p19 gene;(ii) a gene that suppresses the expression of Ink4a/Arf gene; and (iii) a polycomb gene, is further forcibly expressed, it is preferable that a method for producing megakaryocytes further includes a step of arresting the gene forcibly expressed and culturing the resultant cells. In this step, it is preferable that a gene selected from the group consisting of (i) a gene that suppresses the expression of p16 gene or p19 gene;(ii) a gene that suppresses the expression of Ink4a/Arf gene; and (iii) a polycomb gene is forcibly expressed in hematopoietic progenitor cells and thereafter an apoptosis suppression gene is further forcibly expressed in the hematopoietic progenitor cells.

In the present invention, these genes can be forcibly expressed in the hematopoietic progenitor cells by a method known to those skilled in the art, for example, by introducing vectors that expresses these genes or proteins or RNAs encoded by these genes into hematopoietic progenitor cells, or alternatively by bringing, for example, a small-molecule compound that induces expression of these genes into contact with hematopoietic progenitor cells. In the present invention, it is necessary to keep the expression of the genes for a predetermined period. Thus, the introduction of an expression vector, a protein, RNA or a low-molecular compound that induces expression may be repeated a plurality of times such that expression continues during a requisite period.

The vectors that express these genes refer to viral vectors such as a retrovirus, lentivirus, adenovirus, adeno-associated virus, herpes virus and Sendai virus, and plasmids that are expressed in animal cells (e.g., pA1-11, pXT1, pRc/CMV, pRc/RSV, pcDNAI/Neo). To attain continuous expression by single-time introduction, a retrovirus vector or a lentivirus vector is preferable.

Example of the promoter to be used in the expression vector includes EF-α promoter, CAG promoter, SRα promoter, SV40 promoter, LTR promoter, CMV (cytomegalovirus) promoter, RSV (Rous sarcoma virus) promoter, MoMuLV (Moloney mouse leukemia virus) LTR and HSV-TK (herpes simplex virus thymidine kinase) promoter. Other than the promoter, the expression vector, if desired, may contain an enhancer, a poly A addition signal, a selective marker gene and a SV40 replication origin. As a useful selective marker gene, for example, a dihydrofolate reductase gene, a neomycin-resistant gene and a puromycin-resistant gene may be used.

The expression vector of the present invention may be a drug responsive vector having a tetracycline reactive element in the promoter region in order to control expression of a gene by tetracycline or doxycycline. Other than this, an expression vector having loxP sequences, which are arranged so as to sandwich a gene or a promoter region or both of them, may be used in order to excise out the gene from the vector by use of the Cre-loxP system.

In the present invention, in order to simultaneously introduce a plurality of genes, a polycistronic vector in which genes are tandemly linked may be prepared. To enable polycistronic expression, for example, a 2A self-cleavage peptide of a foot-and-mouth disease virus (see, for example, Science, 322, 949-953, 2008) or IRES sequence may be used by ligating these between the genes to be forcibly expressed.

In the present invention, an expression vector may be introduced into hematopoietic progenitor cells by the following methods. In the case of a virus vector, a plasmid containing a desired nucleic acid is introduced into an appropriate packaging cell (e.g., Plat-E cell) or a supplemental cell line (e.g., 293 cell); and viruses produced in culture supernatant are collected and brought into contact with hematopoietic progenitor cells to infect the cells with the viruses. In the case of non-viral vector, a plasmid vector can be introduced into a cell by use of e.g., a lipofection method, a liposome method, an electroporation method, a calcium phosphate coprecipitation method, a DEAE dextran method, a microinjection method or a gene gun method.

In the present invention, in place of forcibly expressing an apoptosis suppression gene in hematopoietic progenitor cells, a caspase inhibitor may be brought into contact with the cells. Examples of caspase inhibitor may be any one of a peptidic compound, a non-peptidic compound and a protein derived from an organism. Example of the peptidic compound may include the following artificially and chemically synthesized peptidic compounds (1) to (10):
(1) Z-Asp-CH2-DCB (molecular weight 454.26)
(2) Boc-Asp (OMe)-FMK (molecular weight 263.3)
(3) Boc-Asp (OBzl)-CMK (molecular weight 355.8)
(4) Ac-AAVALLPAVLLALLAP-YVAD-CHO (molecular weight 1990.5) (SEQ ID No: 1)
(5) Ac-AAVALLPAVLLALLAP-DEVD-CHO (molecular weight 2000.4) (SEQ ID No: 2)
(6) Ac-AAVALLPAVLLALLAP-LEVD-CHO (molecular weight 1998.5) (SEQ ID No: 3)
(7) Ac-AAVALLPAVLLALLAP-IETD-CHO (molecular weight 2000.5) (SEQ ID No: 4)
(8) Ac-AAVALLPAVLLALLAP-LEHD-CHO (molecular weight 2036.5) (SEQ ID No: 5)
(9) Z-DEVD-FMK (Z-Asp-Glu-Val-Asp-fluoromethylketone) (SEQ ID No: 6)
(10) Z-VAD FMK.

Examples of the peptidic compound serving as a caspase inhibitor include
(1) VX-740-Vertex Pharmaceuticals (Leung-Toung et al., Curr. Med. Chem. 9, 979-1002 (2002)) and (2) HMR-3480-Aventis Pharma AG (Randle et al., Expert Opin. Investig. Drugs 10, 1207-1209 (2001)).

Example of the non-peptidic compound serving as the caspase inhibitor includes
(1) anilinoquinazolines (AQZs)-AstraZeneca Pharmaceuticals (Scott et al., J. Pharmacol. Exp. Ther. 304, 433-440 (2003)),
(2) M826-Merck Frosst (Han et al., J. Biol. Chem. 277, 30128-30136 (2002)),
(3) M867-Merck Frosst (Methot et al., J. Exp. Med. 199, 199-207 (2004)), and
(4) Nicotinyl aspartyl ketones-Merck Frosst (Isabel et al., Bioorg. Med. Chem. Lett. 13, 2137-2140 (2003)).

Other examples of the non-peptidic compound serving as the caspase inhibitor include
(1) IDN-6556-Idun Pharmaceuticals (Hoglen et al., J. Pharmacol. Exp. Ther. 309, 634-640 (2004)),
(2) MF-286 and MF-867-Merck Frosst (Los et al., Drug Discov. Today 8, 67-77 (2003)),
(3) IDN-5370-Idun Pharmaceuticals (Deckwerth et al., Drug Dev. Res. 52, 579-586 (2001)),
(4) IDN-1965-Idun Pharmaceuticals (Hoglen et al., J. Pharmacol. Exp. Ther. 297, 811-818 (2001)) and
(5) VX-799-Vertex Pharmaceuticals (Los et al., Drug Discov. Today 8, 67-77 (2003)).

Other than these, e.g., M-920 and M-791-Merck Frosst (Hotchkiss et al., Nat. Immunol. 1, 496-501 (2000)) may be used as the caspase inhibitor.

In the presently claimed invention, the caspase inhibitor is Z-VAD FMK. Z-VAD FMK is used by adding it in a culture medium for culturing hematopoietic progenitor cells. The preferable concentration of Z-VAD FMK in a medium is, for example, 10 µM or more, 20 µM or more, 30 µM or more, 40 µM or more, and 50 µM or more and preferably, 30 µM or more.

In the present invention, as a method for culturing cells in which exogenous genes such as an apoptosis suppression gene is forcibly expressed as mentioned above, a method of culturing the cells on feeder cells using an arbitrary medium may be used. The feeder cells are not particularly limited as long as they can induce megakaryocytes or megakaryocytic progenitor cells and, for example, C3H10T1/2 (Katagiri T, et al., Biochem Biophys Res Commun. 172, 295-299 (1990)) is used.

The medium to be used in the present invention is not particularly limited; however, a medium used for culturing animal cells can be prepared as a fundamental medium. Examples of the fundamental medium include IMDM medium, Medium 199, Eagle's Minimum Essential Medium (EMEM), αMEM medium, Dulbecco's modified Eagle's Medium (DMEM), Ham's F12 medium, RPMI 1640 medium, Fischer's medium, Neurobasal Medium (Life technologies) and a mixed medium of these. In the medium, the serum may be contained or not contained. The medium may contain, if necessary, for example, one or more substances such as albumin, insulin, transferrin, selenium, fatty acids, trace elements, 2-mercaptoethanol, thiol glycerol, lipids, amino acids, L-glutamine, non-essential amino acids, vitamins, growth factors, small molecule compounds, antibiotics, antioxidizers, pyruvic acid, buffers, inorganic salts and cytokines. The cytokines refer to proteins accelerating differentiation of hematocytes such as, for example, VEGF, TPO and SCF A preferable medium in the present invention is IMDM medium containing serum, insulin, transferrin, serine, thiol glycerol, ascorbic acid and TPO, and more preferably further containing SCF. In the case where an expression vector containing a drug responsive promoter is used, during the forced expression step, for example, a corresponding drug such as tetracycline or doxycycline is desirably added to the medium.

In the present invention, culture condition is not particularly limited; however, it was confirmed that differentiation of megakaryocytes or megakaryocytic progenitor cells was accelerated by culturing at a temperature of 37°C or more. Here, the temperature of 37°C or more should be a temperature at which cells are not damaged, and for example, about 37°C to about 42°C or about 37°C to about 39°C is preferable. The culture period at a temperature of 37°C or more can be appropriately determined while monitoring the number of megakaryocytes or megakaryocytic progenitor cells. As long as desired megakaryocytic progenitor cells can be obtained, the number of days is not particularly limited and is, for example, at least 6 days or more, 12 days or more, 18 days or more, 24 days or more, 30 days or more, 42 days or more, 48 days or more, 54 days or more and 60 days or more, and preferably 60 days or more. Long culture period is acceptable in producing megakaryocytes. Furthermore, during the culture period, it is desirable that subcultures are appropriately performed.

According to one aspect of the method for producing megakaryocytes of the present invention, (a) a substance that inhibits the expression or function of p53 gene product, (b) an actomyosin complex function inhibitor, (c) a ROCK inhibitor and (d) an HDAC inhibitor may be further contained in a medium. These methods may be performed in accordance with the method described, for example, in WO2012/157586.

The method for producing a megakaryocyte of the present invention further includes a step of arresting the forced expression in the megakaryocytes or megakaryocytic progenitor cells, which is obtained in a step of forcibly expressing exogenous genes as mentioned above, and culturing the cells. A method for arresting forced expression is as follows. For example, when the forced expression is performed by use of a drug responsive vector, the forced expression may be arrested by preventing contact between the corresponding drug to the cells. In addition, where a vector containing LoxP as mentioned above is used, the forced expression may be arrested by introducing Cre recombinase into the cell. Further, where a temporary expression vector and an RNA or a protein are introduced, the forced expression may be arrested by terminating contact with the vector and others. The medium used in this step may be the same as mentioned above.

Cell culture condition in which the forced expression is arrested is not particularly limited; however, a culture condition of, for example, about 37°C to about 42°C or about 37°C to about 39°C is preferable. When culture is performed at a temperature of 37°C or more, the culture period can be appropriately determined while monitoring the number of megakaryocytes. The culture period is, for example, about 2 days to 10 days, preferably about 3 days to 7 days and desirably at least 3 days. During the culture period, it is desirable that subcultures are appropriately performed.

The megakaryocytes obtained by the aforementioned method are sufficiently matured and efficiently produce CD42b positive and functional platelets. The CD42b positive platelets have high ability to produce thrombus *in vivo* and *in vitro.* The megakaryocytes obtained in the present invention are megakaryocytes having at least an exogenous apoptosis suppression gene and an oncogene integrated in the chromosome; however expression of these genes has been arrested. In the present specification, maturation of megakaryocytes means that megakaryocytes are sufficiently multinucleated and can produce functional platelets. The maturation of megakaryocytes can be confirmed by an increase in expression level of megakaryocyte maturation associated genes such as GATA1, p45 NF-E2 and beta 1-tubulin.

The megakaryocytes and/or megakaryocytic progenitor cells can produce functional platelets even after the cells are cryopreserved and thawed. Thus, the megakaryocytes and/or megakaryocytic progenitor cells produced by the method of the present invention can be distributed in a cryopreserved state.

### (Blood cell composition)

Also described herein is a blood cell composition obtained by differentiation induction of hematopoietic progenitor cells and having a high megakaryocyte content. The "blood cell composition" herein may include not only "megakaryocytes" produced by the method of the present invention but also megakaryocytes prepared by another method or other blood cells.

When hematopoietic progenitor cells are treated by the method of the present invention, differentiation of the hematopoietic progenitor cells into megakaryocytes can be accelerated. Accordingly, if the method of the present invention is applied to hematopoietic progenitor cells differentiated from e.g., pluripotent stem cells, a cell composition having a high content of megakaryocytes can be obtained. Whether the content of megakaryocytes in a blood cell composition is high or not can be determined by those skilled in the art based on their experience or literatures. When hematopoietic progenitor cells are treated by the method of the present invention, the content of megakaryocytes can be increased to at least, 20% or more, 30% or more, preferably, 40% or more, 50% or more, more preferably 80% or more, compared to the case where they are treated by other methods. Accordingly, it is possible to prepare a megakaryocyte population or blood cell population having a high megakaryocyte abundance ratio by the method of the present invention.

The megakaryocytes and others obtained by the method of the present invention can be transplanted by an appropriate method into a living body and are effective to produce functional platelets *in vivo.* Accordingly, the present application describes a therapeutic agent containing the megakaryocytes obtained by the method of the invention.

A problem of shortage of donors and burden of donors in bone marrow transplantation and a problem of *in vivo* production ability of platelets in umbilical cord blood transplantation can be overcome by the megakaryocytes and others obtained by the method of the present invention. Thus, it can be said that the transplantation therapy of the present application is extremely excellent compared to conventional transplantation therapy.

### (Production method of platelets)

The platelet production method according to the present invention is a method for producing platelets *in vitro* from the megakaryocytes obtained by the method of the present invention.

The platelet production method according to the present invention includes a step of culturing the megakaryocytes obtained by the aforementioned method and collecting platelets from a culture.

The culture condition is not limited. Culture may be made for example, in the presence of TPO (10 to 200 ng/mL, preferably about 50 to 100 ng/mL) or in the presence of TPO (10 to 200 ng/mL, preferably about 50 to 100 ng/mL), SCF (10 to 200 ng/mL, preferably about 50 ng/mL) and Heparin (10 to 100 U/mL, preferably about 25U/mL). As long as the function of platelets can be maintained, culture can be continued. As the culture period, a period of 7 to 15 days may be taken as an example.

The culture temperature is not particularly limited as long as the effect of the invention can be obtained. Culture can be performed at a temperature of 35°C to 40°C and preferably 37°C to 39°C.

In the production method of the present invention, a step of culturing megakaryocytes may be performed in serum-free and/or feeder cell-free conditions. Preferably, the megakaryocytes produced by the method of the present invention are cultured in a medium containing TPO. If the platelet production step can be performed in a serum-free and feeder cell-free medium, the obtained platelets hardly cause a problem of immunogenicity when the platelets are used in clinical practice.
Furthermore, if platelets can be produced without feeder cells, since a step of adhering feeder cells is not required, suspension culture can be made in e.g., a flask, reducing a production cost; at the same time, a large-scale production can be suitably made. Note that, when culture is performed without using feeder cells, the culture may be performed in a conditioned medium. The conditioned medium is not particularly limited and can be prepared in accordance with a method known to those skilled in the art. The conditioned medium can be obtained, for example, by appropriately culturing feeder cells and removing the feeder cells from a culture by a filter.

In one aspect of the platelet production method according to the present invention, a ROCK inhibitor and/or an actomyosin complex function inhibitor are added to a medium. As the ROCK inhibitor and actomyosin complex function inhibitor, the same ones as used in the method for producing multinucleated megakaryocytes as mentioned above can be used. As the ROCK inhibitor, for example, Y27632 can be used. As the actomyosin complex function inhibitor, myosin heavy chain II ATPase inhibitor, namely, blebbistatin can be used. A ROCK inhibitor may be added alone. Alternatively, a ROCK inhibitor and an actomyosin complex function inhibitor may be added alone or in combination.

A ROCK inhibitor and/or actomyosin complex function inhibitor may be preferably added in an amount of 0.1 µM to 30 µM and may be added in an amount of, for example, 0.5 µM to 25 µM or 5 µM to 20 µM.

After a ROCK inhibitor and/or actomyosin complex function inhibitor are added, culture may be performed in a period of one to 15 days and may be, for example, 3 days, 5 days and 7 days. Addition of a ROCK inhibitor and/or actomyosin complex function inhibitor enable to further increase the ratio of CD42b positive platelets.

The platelets obtained by the present invention can be administered to patients as a preparation. In administering the platelets, the platelets obtained by the method of the present invention may be stored and formed into a preparation in e.g., a solution containing for example, human plasma, an infusion agent, a citric acid-containing saline, and glucose acetated Ringer's solution as a main agent and/or PAS (platelet additive solution) (Gulliksson, H. et al., Transfusion, 32: 435-440, (1992)). The storage period is from about 3 to 7 days and preferably 4 days. The storage condition is room temperature (20-24°C) and platelets are desirably shaken, stirred during the storage period.

### (Kit for producing megakaryocytes and/or platelets)

Also described herein is a kit for producing megakaryocytes and/or platelets. The kit contains e.g., an expression vector required for expressing e.g., an apoptosis suppression gene, an oncogene and genes (i) to (iii) mentioned above within the cells, and a reagent(s), a medium, serum, supplements such as growth factors (for example, TPO, EPO, SCF, Heparin, IL-6, IL-11) and antibiotics. In addition, for example, when cells derived from pluripotent cells are used, an antibody (for example, antibody against e.g., Flk1, CD31, CD34, UEA-I lectin) for recognizing a marker used in identifying a net-like structure prepared from these cells is also included. Furthermore, in order to select hematopoietic progenitor cells suitable for producing megakaryocytes, a kit for measuring expression of KLF1 and/or FLI1 may be contained. The reagent(s) and antibodies and others contained in the kit are placed in a container made of a material, which can effectively maintain the activities of these components for a long time and does not adsorb to them or denature them.

The "cells" described in the present specification may be those derived from a human and non-human animals (for example, mouse, rat, cow, horse, pig, sheep, monkey, dog, cat, bird) and are not particularly limited. Particularly preferably, cells derived from a human are used.

Now, the present invention will be more specifically described by way of Example; however, the invention is not limited by Example.

### Example 1 (including reference material)

### 1) Preparation of hematopoietic progenitor cells from ES (reference example) and iPS cells.

Human ES cells (khES3: obtained from Kyoto University) (reference example and not within the scope of the invention) and iPS cells (TKDN SeV2: iPS cells derived from human fetus dermal fibroblasts established by use of Sendai virus; and 585A1, 585B1, 606A1, 648B1 and 692D2: iPS cells derived from human peripheral blood mononuclear cells established by use of episomal vector described in Okita K, et al., Stem Cells 31, 458-66, 2012) were subjected to culture in accordance with the method described in Takayama N., et al. J Exp Med. 2817-2830 (2010) and differentiated into hematocytes. More specifically, human ES/iPS cell colonies were co-cultured with C3H10T1/2 feeder cells in the presence of 20 ng/mL VEGF (R&D SYSTEMS) for 14 days to prepare Hematopoietic Progenitor Cells (HPCs)). The culture condition was 20% O₂, 5% CO₂ (hereinafter, the culture condition was the same unless otherwise specified).

### 2) Viral infection of hematopoietic progenitor cells

Onto a 6-well plate on which C3H10T1/2 feeder cells were previously seeded, HPCs obtained by the aforementioned method were plated (5 × 10⁴ cells/well) and c-Myc and BCL-xL were forcibly expressed by a lentivirus method. At this time, 6 wells were used per cell line. More specifically, viral particles were added to a medium so as to obtain an MOI of 20 to infect the cells with the virus by spin infection (32°C, centrifugation at 900 rpm for 60 minutes). This operation was repeated twice every 12 hours. At this time, a medium (hereinafter, differentiation medium), which was prepared by adding 50 ng/mL Human thrombopoietin (TPO) (R&D SYSTEMS), 50 ng/mL Human Stem Cell Factor (SCF) (R&D SYSTEMS) and 2 µg/mL Doxycyclin (Dox) to a basal medium (Iscove's Modified Dulbecco's Medium (IMDM) (Sigma-Aldrich) containing 15% Fetal Bovine Serum (GIBCO), 1% Penicillin-Streptomycin-Glutamine (GIBCO), 1% Insulin, Transferrin, Selenium Solution (ITS-G) (GIBCO), 0.45mM 1-Thioglycerol (Sigma-Aldrich) and 50 µg/mL L-Ascorbic Acid (Sigma-Aldrich)) was used. Note that, lentivirus vectors, which are inducible vectors controlled by Tetracycline, were prepared by replacing an mOKS cassette of LV-TRE-mOKS-Ubc-tTA-I2G (Kobayashi, T., et al. Cell 142, 787-799 (2010)) with Bcl-xL and c-Myc (LV-TRE-BCL-xL-Ubc-tTA-I2G and LV-TRE-c-Myc-Ubc-tTA-I2G, respectively). The viral particles used for infection were prepared by expressing the above lentivirus vectors in 293T cells.

### 3) Establishment of megakaryocytic cell line and maintenance of culture

The day on which viral infection was performed was defined as Day 0 from Infection. A megakaryocytic cell line was established by culturing as follows.

### • Day 2 from infection: Subculture

The cells infected with the viruses in accordance with the aforementioned method were collected by pipetting and centrifuged at 1200 rpm for 5 minutes. After the supernatant was removed, the cell pellet was suspended in a fresh differentiation medium and plated on fresh C3H10T1/2 feeder cells (6-well plate).

### • Day 6 from infection: Subculture

The same operation as in Day 2 from infection was repeated. Note that an establishment operation was performed a plurality of times. First one of the establishment operations (Exp.1) was performed in a differentiation medium without SCF. The impression that proliferation of cells was good in the presence of SCF was obtained.

### • Day 12 from infection: Subculture

The same operation as in Day 6 from infection was performed. After the number of cells was counted, the cells were seeded (3 × 10⁵ cells/10 mL/100 mm dish).

### • Day 18 from infection: Subculture

The same operation as in Day 6 from infection was performed. After the number of cells was counted, the cells were seeded (3 × 10⁵ cells/10 mL/100 mm dish).

### • Day 24 from infection: Subculture, cryopreservation, FACS analysis.

Part of the cells was taken and subcultured (1 × 10⁵ cells/well) in the same as in the above. The remainder was cryopreserved (about 5 × 10⁵ cells/tube).

Thereafter, subculture was performed every 4 to 7 days and maintenance culture was performed. During this period, the medium was not exchanged with a fresh one.

### 4) Analysis of megakaryocytic cell line

Megakaryocytic cell line was tried to be established from the hematopoietic progenitor cells derived from ES cells (khES3) (reference example) and iPS cells (TKDN SeV2) by the aforementioned method. As a result, it was confirmed that a megakaryocytic cell line was established from the hematopoietic progenitor cells derived from TKDN SeV2 in three out of six samples; however, in the hematopoietic progenitor cells derived from KhES3, a megakaryocytic cell line was not established in six samples (Figure 1A).

Note that, establishment of megakaryocytic cell line was determined in accordance with the following method. On Day 24 from infection, hematocytes were collected. The hematocytes (1.0 × 10⁵ cells) were immuno-stained with anti-human CD41a-APC antibodies (BioLegend), anti-human CD42b-PE antibodies (eBioscience) and anti-human CD235ab-pacific blue antibodies in an amount of 2 µL, 1 µL, and 1 µL, respectively and thereafter analyzed by FACSAria™II cell sorter (BD) to check establishment of a megakaryocytic cell line. Since the number of megakaryocytes derived from iPS cells usually decreases on and after Day 10 in this differentiation system (Takayama N., et al. J Exp Med. 2817-2830 (2010)), establishment of the megakaryocytic cell line was confirmed based on the fact that CD41a+ cells continuously proliferated even on Day 24 from infection.

Subsequently, a megakaryocytic cell line was established using hematopoietic progenitor cells derived from the iPS cells (TKDN SeV2), in the same manner, and the wells in which infected cells continuously proliferated was selected and the number of cells was counted (Figure 1A). It was confirmed that in the cell line containing a large number of adhesion cells, the cell proliferation rate was relatively low. It was also confirmed that the cell line can be subcultured for at least 40 days.

Since a megakaryocytic cell line was not established from ES cell-derived hematopoietic progenitor cells, (reference example), KLF1 and FLI1 genes were analyzed in ES cell- and iPS cell-derived hematopoietic progenitor cells by StepOnePlus™ real time PCR system (Applied Biosystems). As a result, difference in expression of these genes was found (Figure 1B). From this, it was suggested that megakaryocytes are easily established from the hematopoietic progenitor cells in which KLF1 expression is low or from the hematopoietic progenitor cells in which FLI1 expression is high.

### 5) Maturation of megakaryocytes by arresting expression of introduced gene

On Day 24 from infection, cells (5.0 × 10⁵ cells/well) of the megakaryocytic cell line was cultured on C3H10T1/2 feeder cells by use of a differentiation medium (in two conditions: in presence and absence of SCF) containing or not containing Dox for 3 or 5 days. The former is the condition that the introduced genes are expressed (Gene-ON) and the latter is the condition that expression of the introduced genes is arrested (Gene-OFF), respectively. A culture solution was collected by pipetting and subjected to analysis for a cell proliferation rate (Figure 2), FACS analysis of hematocytes fraction and platelets fraction (Figures 3 and 4) and gene expression analysis (Figure 5). FACS analysis was performed in the same manner as above. The gene expression analysis was performed in accordance with a customary method. More specifically, RNA was extracted, cDNA was prepared and genes were analyzed by use of universal probes or taqman probes. The genes analyzed were GAPDH, c-Myc, Bcl-xL, GATA1, p45 NF-E2, betal-tubulin and c-MPL.

As a result, it was confirmed that expression levels of introduced genes, c-Myc and Bcl-xL, significantly decreased in the conditions of Gene-OFF and cell proliferation was arrested. In accordance with this, it was found that expression levels of genes associated with megakaryocyte maturation (GATA1, p45 NF-E2, beta 1-tubulin) significantly increased. Furthermore, in connection with a change in expression levels of these genes, the expression levels of the megakaryocytic cell line and CD42b on platelets increased. As described above, it was suggested that maturation of the megakaryocytic cell line established from hematopoietic progenitor cells was accelerated by arrest of expression of the introduced genes.

### 6) Function test of megakaryocyte

The megakaryocytic cell line (Day 40 from initiation of culture) prepared from iPS cells (TKDN SeV2) by the aforementioned method and the megakaryocytes (Day 21 from initiation of differentiation induction) prepared from ES cells (khES3) by the method described in Takayama et al., Blood, 111: 5298-5306 2008 (reference example) were stimulated with Phorbol 12-Myristate 13-acetate (PMA). Immediately after the stimulation, the binding ability of them to Fibrinogen was measured (Figure 6). As a result, it was confirmed that the megakaryocytic cell line prepared by the method of the present invention had a binding ability to Fibrinogen in response to PMA stimulation; however, the megakaryocytes obtained by the method known in the art did not have a significant Fibrinogen binding ability even after the megakaryocytes were stimulated with PMA. From the above, it was suggested that in the megakaryocytes prepared by the method of the present invention, more matured megakaryocytes can be obtained.

### Example 2 (including reference material)

### 1) Induction of megakaryocytic progenitor cells that can be proliferated in expanding culture by use of c-MYC and BMI1

To KhES3-derived HPCs obtained by the method described in Example 1 (reference example and not within the scope of the invention) (1) c-Myc alone, (2) Bmi1 alone, (3) c-MYC and sh-p53, (4) c-MYC and BCL-XL, (5) c-MYC and sh-ARF, (6) c-MYC and BMI1, or (7) c-MYC, sh-INK4A and sh-ARF was introduced by use of a single retrovirus vector per gene. The resultant HPCs were cultured in a basal medium supplemented with 50 ng/mL TPO and 50 ng/mL SCF. In the cases where at least c-MYC was introduced, megakaryocytic progenitor cells of CD41a+, CD42a+, CD42b+ and CD9+ were obtained. The culture was further continued. As a result, HPCs having (6) c-MYC and BMI1, and (7) c-MYC, sh-INK4A and sh-ARF were successfully proliferated in expanding culture continuously for two months (Figure 7A). The retrovirus vectors used for introduction of genes were pMXs retro-vector (see, Takahashi K, et al., Cell.;131: 861-872, 2007 or Ohmine K, et al., Oncogene 20, 8249-8257, 2001) and pGCDNsam retro-vector (received from Prof. Iwama of Chiba University). Sh-p53 gene was prepared with reference to Brummelkamp TR, et al., Science 296, 550-553, 2002, whereas sh-INK4A and sh-ARF were prepared with reference to Voorhoeve PM and Agami R, Cell 4, 311-319, 2003.

The megakaryocytic progenitor cells obtained had basophilic monoblastoid morphology (Figure 7B) and produced abnormal platelet-like particles of CD41a-positive and having relatively low CD42b expression. This is conceivably caused because forced expression of c-Myc was maintained.

### 2) Confirmation of significance of c-MYC expression level

c-MYC and BMI1 were forcibly expressed by using the construct shown in Figure 7C, i.e., a retrovirus vector having c-MYC-2A-BMI1 or BMI1-2A-c-MYC, and megakaryocytic progenitor cells were induced in the same culture condition as above. As a result, only in the case where c-MYC-2A-BMI1 was used, expanding culture of 40 days or more was successfully made (Figure 7D). Expression levels of c-Myc in each introduction method were checked. As a result, it was confirmed that in the case where c-MYC-2A-BMI1 was used, the expression level of c-MYC was lower than in the case where BMI1-2A-c-MYC was used (Figure 7E). Therefore, in order to suppress expression of c-Myc, a c-MYC expression vector having a destabilization domain (DD) at the C-terminal was used. The expression vector having DD, i.e., a vector expressing c-MYC-DD-2A-BMI1 was constructed by use of pPTunerC vector and Shied-1 (Clontech/Takara Bio). This c-MYC-DD-2A-BMI1 expression vector was introduced into HPCs in the same manner as above and culture was continued. As a result, CD41a-positive megakaryocytic progenitor cells were successfully proliferated in expanding culture for at least 50 days (Figure 8A). In contrast, in the case of c-MYC-2A-BMI1, expanding culture was not able to be successfully maintained. Since, when the expression of c-MYC was stabilized by adding Shield-1, the number of megakaryocytic progenitor cells decreased in volume-dependent manner, it was confirmed that Shield-1 did not have toxic effect and that expression level of c-MYC influenced expanding culture (Figure 8B). The effect of c-MYC expression on the expanding culture is predicted due to a caspase-dependent apoptosis. Therefore, the activity of caspase-3/7 in the presence of Shield-1 was then measured. As a result, it was confirmed that caspase was activated along with stabilization of c-MYC (Figure 8C). Based on the above, it was suggested that apoptosis occurs by overexpression of c-MYC and inhibits expanding culture of megakaryocytic progenitor cells.

### 3) Induction of megakaryocytic progenitor cells by suppression of caspase activity due to BCL-XL expression

Megakaryocytic progenitor cells can be induced by forced expression of c-MYC and BMI1; however, apoptosis occurs depending upon the expression level of c-MYC and limits the expanding culture. Then, to suppress apoptosis, BCL-XL was introduced 14 days to 21 days after c-MYC and BMI1 were introduced (Figure 9A). As a result, it was confirmed that megakaryocytic progenitor cells induced from iPS cell-derived HPCs (Cl-1: derived from 692D2 line) and ES cell-derived HPCs (Cl-2: derived from khES3 (reference example) were able to be successfully proliferated in expanding culture for 5 months or more (Figures 9B and C). Furthermore, c-MYC-DD, BMI1 and BCL-XL were simultaneously expressed in HPCs and the expression level of c-MYC was controlled by varying the addition amount of Shield-1. In this condition, the number of megakaryocyte cells was counted on Day 7. It was confirmed that even when the expression level of c-MYC was high, if BCL-XL was expressed, megakaryocytic progenitor cells were induced (Figures 9D and E).

In place of expressing BCL-XL, another method for controlling caspase was investigated as follows. After c-Myc and BMI1 were introduced, a caspase inhibitor, i.e., Z-VAD FMK (Merck) (10 µM or 30 µM) was added. In this condition, culture was continued for 66 days. When BCL-XL was expressed, the cells proliferated by 64 times; whereas when 30 µM of Z-VAD FMK was added, the cells proliferated by 21 times (Figure 10A). In contrast, when DMSO was used (negative control), no proliferation was observed. Furthermore, when DMSO was used, the number of Annexin V positive cells was counted. As a result, many Annexin V positive cells were present (66.5%). From this, it was confirmed that apoptosis was not suppressed.

Subsequently, timing of introducing BCL-XL was investigated, four types of iPS cell clones were induced into megakaryocytic progenitor cells (Cl-3: derived from KhES3 line, Cl-4: derived from 692D2 line, Cl-6: derived from 585A1 line and Cl-7: derived from TKDN SeV2 line) in accordance with the following two protocols: (1) simultaneously introducing BCL-XL, c-MYC and BMI1 and (2) introducing Bmi1 and c-MYC and, 14 days to 21 days later, introducing BCL-XL . When the protocol of (1) simultaneous introduction was used, megakaryocytic progenitor cells were able to be proliferated up to 40 days to 50 days in expanding culture, for any one of the iPS cell clones. In contrast, when the protocol of (2) expressing BCL-XL later, megakaryocytic progenitor cells were able to be proliferated in expanding culture continuously for 60 days or more, for any one of ES cell clones (reference example) or iPS cell clones (Figures 10B, C, D and E).

In order to investigate karyotype variation in long term culture, megakaryocytic progenitor cells (Cl-1, Cl-2 and Cl-7) derived from three types of iPS cell clones were continuously cultured for 5 months and then karyotype was analyzed. As a result, the karyotype of megakaryocytic progenitor cells (Cl-7) only was normal. These three types of megakaryocytic progenitor cells (2 × 10⁶) were intravenously injected to immunodeficiency mice (n = 5) to which no radiation was applied, and monitored for 16 weeks or 20 weeks. As a result, the case where one (Cl-2) of the two types of megakaryocytic progenitor cells having karyotype abnormality was injected, leukemia was induced and mice died in early time (Figure 10F). However, in Cl-7 to which the method of the present invention was applied, it was confirmed that megakaryocytic progenitor cells that showes normal karyotype and induces no leukemia *in vivo* when injected were able to be induced.

### 4) Cryopreservation/thaw of induced megakaryocytic progenitor cell

After the megakaryocytic progenitor cell line prepared by a method of introducing c-MYC and BMI1 and thereafter introducing BCL-xL as mentioned above was cryopreserved, thawed and cultured in the same condition, the megakaryocytic progenitor cell line was successfully proliferated in expanding culture for 21 days (Figure 11A). When the expression of cell markers was checked at this time, expression levels of CD41a, CD42a, CD42b and CD9 were the same as those before cryopreservation (Figure 11B). Accordingly, it was demonstrated that the megakaryocytic progenitor cells produced by the method of the present invention can be cryopreserved.

### 5) Maturation step of induced megakaryocytic progenitor cells

Expression of exogenous genes, c-MYC, BMI1 and BCL-XL, in the megakaryocytic progenitor cells obtained by the aforementioned method was arrested by exchanging the medium with a medium containing no Dox and the megakaryocytic progenitor cells was cultured for 5 days (Figure 12A). As a result, multinucleation was observed in 20.2% of the cells (Figure 12B). At this time, it was confirmed that CD42b-positive proplatelets were formed. On Day 4 after arrest of expression, in megakaryocytic progenitor cells (Cl-2 and Cl-7) derived from two types of clones, expression level of CD42b increased (indicating maturation) as shown in Figure 12C. With maturation from megakaryocytic progenitor cells to megakaryocytes, expression levels of GATA1, FOG1, NF-E2 and β1-tubulin were confirmed to increase.

### 6) Induction of CD41a-positive and CD42b-positive platelets

As described above, CD42b expression was increased by arresting expression of exogenous genes, c-MYC, BMI1 and BCL-XL, and CD41a-positive and CD42b positive platelets were obtained (Figure 13A). Then, which expression of exogenous genes must be arrested to produce platelets most efficiently was investigated. The case where expression of BCL-XL alone was maintained was compared to the case where expression of all three genes was arrested. As a result, it was found that platelets were most efficiently produced in the case where expression of all three genes was arrested (Figure 13B). Furthermore, conventional megakaryocytic cell lines (Meg01 (ATCC), CMK and K562 (received from Dr. H. Kashiwagi of Osaka University)) were cultured in RPMI supplemented with 10% FBS and PSG. The productions of CD41a-positive and CD42b positive particles from the conventional megakaryocytic cell lines in the medium to which 100 nM PMA was added were compared to the production of the particles from iPS cell-derived megakaryocytic progenitor cells. As a result, it was found that the amount of platelets produced from the iPS cell-derived megakaryocytic progenitor cells was significantly large (Figure 13C). At this time, a large number of CD41a-positive and CD42b negative platelets-like particles were produced from the conventional megakaryocytic cell line. Subsequently, megakaryocytic progenitor cells in which forced expression of exogenous genes was arrested were cultured for 5 days in a serum-free medium and the amount of CD42b positive platelets produced from a single induced megakaryocytic progenitor cell was measured. As a result, three platelets were obtained from a single cell in Cl-2 and 10 platelets in Cl-7. Similarly, megakaryocytic progenitor cells in which forced expression of exogenous genes was arrested were cultured in a 10 cm dish (10 mL medium) to produce platelets. As a result, platelets of 4 × 10⁶ (Cl-7) and 2 × 10⁶ (Cl-2) were produced per medium (1 mL) (Figure 13D). From this, it was suggested that 10¹¹ of platelets, which were required for a single platelet transfusion, could be produced by culturing megakaryocytic progenitor cells in a 25 to 50L medium.

The platelets (hereinafter, imMKCL platelets) obtained by the above method (expression of exogenous genes was arrested and cultured in serum-free medium for 5 days) were observed by a scanning electron microscope. The microtubule looked normal; however, the number of granules was relatively low compared to the human platelets (hereinafter, Fresh platelets) taken by blood sampling (Figure 14A). Further, in order to confirm the function of imMKCL platelets, platelets were extracted in the same method as described in De Cuyper, IM, et al., Blood 121,e70-80, 2013 and stimulated by 1 U/mL thrombin and 200 µM ADP. Then, the binding ability of the platelets to PAC-1 was measured by a flow cytometer. As a result, it was found that imMKCL platelets bind to PAC-1 in response to the stimulation (Figures 14B and C). The binding ability was inferior to that of human platelets obtained by blood sampling but higher than the platelets pooled at 37°C for 5 days (hereinafter, pooled platelet). Furthermore, imMKCL platelets or Fresh platelets were mixed with the same number of Fresh platelets. To the mixture, 100 µM ADP and 100 µM TRAP6, or 10 µg/mL collagen (Nycomed) were added. The resultant mixture was shaken at 37°C for 10 minutes to stimulate the platelets and thereafter agglutination of platelets was checked. As a result, even in imMKCL platelets, platelet agglutination was observed (Figures 14D and E). In addition, when imMKCL platelets were added to IMDM containing 20% platelet free plasma and the Clot test (coagulation test) was performed by stimulating the platelets with 2 U/mL thrombin, the imMKCL platelets coagulated in response to stimulation with thrombin. After imMKCL platelets were stimulated with thrombin, 100 nM PMA and 10 µg/mL collagen, ADP-releasing ability was checked by use of EnzyLightTM ADP Assay Kit (BioAssay System) and von Willebrand factor (vWF)-releasing ability was checked by use of Von Willebrand factor Human ELISA Kit (Abcam). As a result, it was confirmed that the imMKCL platelets had these releasing abilities, which were inferior to Fresh platelets. From the above, it was confirmed that the function of the imMKCL platelets was lower than that of Fresh platelets but higher than that of the pooled platelets.

The function of imMKCL platelets *ex-vivo* was checked as follows. To a chamber coated with 10 µg/mL vWF, platelets were flowed through microchannel (Ibidi) at a flow rate of 1600s-1. As a result, 62.3% of Fresh platelets (derived from Cl-2) and 75.8% of imMKCL platelets (derived from Cl-7) were bound to the channel. Since the binding function was suppressed by adding anti-CD42b (HIP1) (Abcam), it was shown that the binding function is a CD42b dependent function (Figures 14F and G) .

### 7) Thrombogenic activity of induced platelets in thrombocytopenia model mouse

To NOG mice (thrombocytopenia model mouse) at Day 9 after 2.4 Gy of radiation was applied, Fresh platelets (1 × 10⁸) or imMKCL platelets (6 × 10⁸ or 1 × 10⁸) were administered through the tail vein. Blood (50 to 100 µL) was sampled 30 minutes, 2 hours and 24 hours after the administration and the number of human CD41a-positive platelets was counted (Figure 15A). As a result, no significant difference was observed in the reduction rate of human CD41a-positive platelets between imMKCL platelets and Fresh platelets, in a mouse body.

To a thrombocytopenia model mouse, laser was applied to prepare a blood vessel damage model mouse. Thrombus formation was observed in the model mouse by a high spatial and temporal resolution confocal microscope while maintaining blood flow without breaking the endodermis. As a result, it was found that thrombus was formed in the laser irradiation site in either case where Fresh platelet and imMKCL platelets were administered. At this time, it was found that one of the human derived platelets adhered to the blood vessel without agglutinated with mouse platelets (Figure 15C). It was further found that such adhesion to a blood vessel was suppressed by administering AK4 antibody (anti-P-selectin antibody) (Figure 15D). This suggests that imMKCL platelets initially adhere to a blood vessel wall depending upon P-selectin. The same test was repeated with respect to imMKCL platelets prepared from four types of iPS cell clones. As a result, it was found that imMKCL platelets can be much more participated in thrombus formation than the pooled platelet (Figures 15E and F).

As described above, megakaryocytic progenitor cells can be proliferated in expanding culture and cryopreserved. Owing to this, a requisite number of megakaryocytic progenitor cells can be proliferated from pluripotent stem cells and stored, with the result that requisite platelets can be prepared for only 5 days. Furthermore, since the megakaryocytic progenitor cells are proliferated by expanding culture, a culture solution required for producing an intermediate, i.e., HPCs, is saved and cost required for preparing platelets can be reduced.

## Claims

1. A method for producing megakaryocytes from hematopoietic progenitor cells, comprising the following (i) and (ii) steps:
(i) forcibly expressing BCL-XL gene and c-MYC gene in hematopoietic progenitor cells and culturing the cells, and
(ii) arresting forced expression of the BCL-XL gene and the c-MYC gene in the cells obtained in the step (i) and culturing the cells.

2. The method according to Claim 1, wherein, in the step (i), BMI1 is further forcibly expressed in the hematopoietic progenitor cells; and in the step (ii), the forced expression of the BMI1 is arrested and the culture is performed.

3. The method according to Claim 2, wherein, the step (i) is a step of forcibly expressing the c-MYC gene and the BMI1 in a hematopoietic progenitor cells, and thereafter, further forcibly expressing the BCL-XL gene in the cells.

4. The method according to Claim 3, wherein, in the step (i), the c-MYC gene and the BMI1 are forcibly expressed in the hematopoietic progenitor cell and the cell is cultured, and thereafter, further the BCL-XL gene is forcibly expressed in the cells.

5. The method according to any one of Claims 1 to 4, wherein, in the steps (i) and (ii), the cells are cultured on C3H10T1/2 cells in (a) a culture solution containing TPO or (b) a culture solution containing TPO and SCF.

6. The method according to any one of Claims 1 to 5, wherein the forced expression of the genes is performed by a drug responsive vector.

7. The method according to any one of Claims 1 to 6, wherein the hematopoietic progenitor cells are (a) cells differentiation-induced from pluripotent stem cells or (b) cells which are differentiation-induced from pluripotent stem cells by culturing the pluripotent stem cells on C3H10T1/2 cells in a culture solution containing VEGF, in the differentiation induction.

8. The method according to any one of Claims 1 to 7, wherein, in the hematopoietic progenitor cells, the expression of KLF1 is low or the expression of FLI1 is high

9. The method according to any one of Claims 1 to 8, wherein the step (ii) is performed for 5 days.

10. A method for producing megakaryocytes from hematopoietic progenitor cells, comprising the following (i) and (ii) steps:
(i) forcibly expressing c-MYC gene in hematopoietic progenitor cells and culturing the cells in a medium containing Z-DEVD-FMK, and
(ii) arresting forced expression of the c-MYC gene in the cells obtained in the step (i) and culturing the cells.

11. A method for producing platelets, comprising recovering platelets from the culture of the megakaryocytes obtained by the method according to any one of Claims 1 to 10.

## Patentansprüche

1. Verfahren zur Herstellung von Megakaryozyten aus hämatopoetischen Vorläuferzellen, das die folgenden Schritte (i) und (ii) umfasst:
(i) das erzwungene Exprimieren des BCL-XL-Gens und des c-MYC-Gens in hämatopoetischen Vorläuferzellen und Kultivieren der Zellen und
(ii) das Anhalten der erzwungenen Expression des BCL-XL-Gens und des c-MYC-Gens in den in Schritt (i) erhaltenen Zellen und das Kultivieren der Zellen.

2. Verfahren nach Anspruch 1, wobei in Schritt (i) die Expression von BMI1 in hämatopoetischen Vorläuferzellen weiter erzwungen wird; und in Schritt (ii) die erzwungene Expression von BMI1 angehalten und die Kultivierung durchgeführt wird.

3. Verfahren nach Anspruch 2, wobei Schritt (i) ein Schritt des erzwungenen Exprimierens des c-MYC-Gens und von BMI1 in hämatopoetischen Vorläuferzellen und danach des weiteren erzwungenen Exprimierens des BCL-XL-Gens in den Zellen ist.

4. Verfahren nach Anspruch 3, wobei das c-MYC-Gen und BMI1 in Schritt (i) in der hämatopoetischen Vorläuferzelle erzwungen exprimiert werden und die Zelle kultiviert wird und das BCL-XL-Gen danach weiter in den Zellen erzwungen exprimiert wird.

5. Verfahren nach einem der Ansprüche 1 bis 4, wobei die Zellen in den Schritten (i) und (ii) auf C3H10T1/2-Zellen in (a) einer Kulturlösung, die TPO enthält, oder (b) einer Kulturlösung, die TPO und SCF enthält, kultiviert werden.

6. Verfahren nach einem der Ansprüche 1 bis 5, wobei die erzwungene Expression der Gene mit einem auf Arzneimittel ansprechenden Vektor durchgeführt wird.

7. Verfahren nach einem der Ansprüche 1 bis 6, wobei die hämatopoetischen Vorläuferzellen (a) Zellen, die aus pluripotenten Stammzellen durch Differenzierung induziert sind, oder (b) Zellen, die aus pluripotenten Stammzellen durch das Kultivieren der pluripotenten Stammzellen auf C3H10T1/2-Zellen in einer Kulturlösung, die VEGF enthält, durch Differenzierung induziert sind, bei der Differenzierungsinduktion sind.

8. Verfahren nach einem der Ansprüche 1 bis 7, wobei in den hämatopoetischen Vorläuferzellen die Expression von KLF1 niedrig oder die Expression von FLI1 hoch ist.

9. Verfahren nach einem der Ansprüche 1 bis 8, wobei Schritt (ii) 5 Tage lang durchgeführt wird.

10. Verfahren zur Herstellung von Megakaryozyten aus hämatopoetischen Vorläuferzellen, das die folgenden Schritte (i) und (ii) umfasst:
(i) das erzwungene Exprimieren des c-MYC-Gens in hämatopoetischen Vorläuferzellen und Kultivieren der Zellen in einem Medium, das Z-DEVD-FMK enthält, und
(ii) das Anhalten der erzwungenen Expression des c-MYC-Gens in den in Schritt (i) erhaltenen Zellen und das Kultivieren der Zellen.

11. Verfahren zur Herstellung von Thrombozyten, das das Gewinnen von Thrombozyten aus der Kultur der Megakaryozyten, die durch ein Verfahren nach einem der Ansprüche 1 bis 10 erhalten wurden, umfasst.

## Revendications

1. Procédé de production de mégacaryocytes à partir de cellules progénitrices hématopoïétiques, comprenant les étapes (i) et (ii) suivantes :
(i) une expression de manière forcée du gène BCL-XL et du gène c-MYC dans des cellules progénitrices hématopoïétiques et la culture de ces cellules, et
(ii) l'arrêt de l'expression forcée du gène BCL-XL et du gène c-MYC dans les cellules obtenues à l'étape (i) et la culture des cellules.

2. Procédé selon la revendication 1, dans lequel, dans l'étape (i), du BMI1 est en outre exprimé de manière forcée dans les cellules progénitrices hématopoïétiques ; et dans l'étape (ii), l'expression forcée du BMI1 est arrêtée et la culture est effectuée.

3. Procédé selon la revendication 2, dans lequel l'étape (i) est une étape consistant à exprimer de manière forcée le gène c-MYC et le BMI1 dans des cellules progénitrices hématopoïétiques, puis en outre à exprimer de manière forcée le gène BCL-XL dans les cellules.

4. Procédé selon la revendication 3, dans lequel, à l'étape (i), le gène c-MYC et le BMI1 sont exprimés de manière forcée dans la cellule progénitrice hématopoïétique et la cellule est cultivée, puis le gène BCL-XL est en outre exprimé de manière forcée dans les cellules.

5. Procédé selon l'une quelconque des revendications 1 à 4, dans lequel, dans les étapes (i) et (ii), les cellules sont cultivées sur des cellules C3H10T1/2 dans (a) une solution de culture contenant de la TPO ou (b) une solution de culture contenant de la TPO et du SCF.

6. Procédé selon l'une quelconque des revendications 1 à 5, dans lequel l'expression forcée des gènes est effectuée par un vecteur sensible à un médicament.

7. Procédé selon l'une quelconque des revendications 1 à 6, dans lequel les cellules progénitrices hématopoïétiques sont (a) des cellules induites par différenciation à partir de cellules souches pluripotentes ou (b) des cellules qui sont induites par différenciation à partir de cellules souches pluripotentes en cultivant les cellules souches pluripotentes sur des cellules C3H10T1/2 dans une solution de culture contenant du VEGF, dans l'induction de différenciation.

8. Procédé selon l'une quelconque des revendications 1 à 7, dans lequel, dans les cellules progénitrices hématopoïétiques, l'expression de KLF1 est faible ou l'expression de FLI1 est élevée.

9. Procédé selon l'une quelconque des revendications 1 à 8, dans lequel l'étape (ii) est effectuée pendant 5 jours.

10. Procédé de production de mégacaryocytes à partir de cellules progénitrices hématopoïétiques, comprenant les étapes (i) et (ii) suivantes :
(i) une expression de manière forcée du gène c-MYC dans des cellules progénitrices hématopoïétiques et une culture des cellules dans un milieu contenant Z-DEVD-FMK, et
(ii) un arrêt de l'expression forcée du gène c-MYC dans les cellules obtenues à l'étape (i) et une culture des cellules.

11. Procédé de production de plaquettes, comprenant la récupération de plaquettes à partir de la culture des mégacaryocytes obtenus par le procédé selon l'une quelconque des revendications 1 à 10.
